# EUROPÄISCHE PATENTANMELDUNG

(11) **EP 0 051 773 A1**
(43) Veröffentlichungstag der Anmeldung: **19.05.1982**
(21) Anmeldenummer: 81108760.0
(22) Anmeldetag: 23.10.1981
(51) Int. Cl.: C07D 499/64, A61K 31/43, C07D 239/48, C07D 409/12

(54) **Neue Penicilline, ihre Salze, Verfahren zu ihrer Herstellung und diese Verbindungen enthaltende Arzneimittel**

(30) Priorität: 11.11.1980 DE 3042440
(71) Anmelder: Dr. Karl Thomae GmbH, D-88397 Biberach (DE)
(72) Erfinder: Wetzel, Bernd, Dr. Dipl.-Chem., D-7950 Biberach 1 (DE); Woitun, Eberhard, Dr. Dipl.-Chem., D-7950 Biberach 1 (DE); Reuter, Wolfgang, Dr. Dipl.-Chem., D-7951 Laupertshausen (DE); Maier, Roland, Dr. Dipl.-Chem., D-7950 Biberach 1 (DE); Lechner, Uwe, Dr., D-7951 Ummendorf (DE); Goeth, Hanns, Dr., D-7950 Biberach 1 (DE)

(57) **Zusammenfassung**

Es werden neue Penicilline der allgemeinen Formel 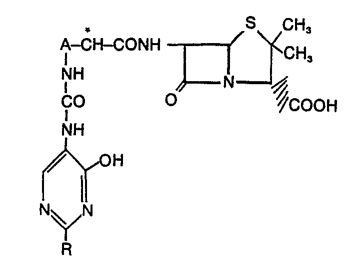 und deren Salze beschrieben, worin A die Phenylgruppe die p-Hydroxyphenylgruppe, die 2-Thienylgruppe oder die Cyclohexa-1,4-dien-1-yl-, 3,4-Dihydroxyphenyl- oder 3-Chlor-4-hydroxyphenylgruppe und R Gruppen der Formeln 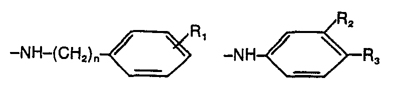 bedeuten, worin R, u.a. die Ureidogruppe, die Nitrogruppe, eine gegebenenfalls durch Methylreste substituierte Aminocarbonylgruppe, die Acetylgruppe oder Gruppen der Formeln -SO₂R₄; -SOR₄; -SO₂NH₂; -SO₂-NHR₄; -SO₂N(R₄)₂ und R₂ und/oder R₃ die Hydroxylgruppe, die Aminosulfonylgruppe, die Aminocarbonyl- oder Methylaminocarbonylgruppe darstellen.

Es werden des weiteren zwei Wege zur Herstellung der Verbindungen der allgemeinen Formel I angegeben. Die Verbindungen der allgemeinen Formel I zeigen gegen grampositive, insbesondere aber gegen gramnegative Bakterien eine sehr starke Wirkung.

## Beschreibung

Die Erfindung betrifft neue Penicilline der allgemeinen Formel I 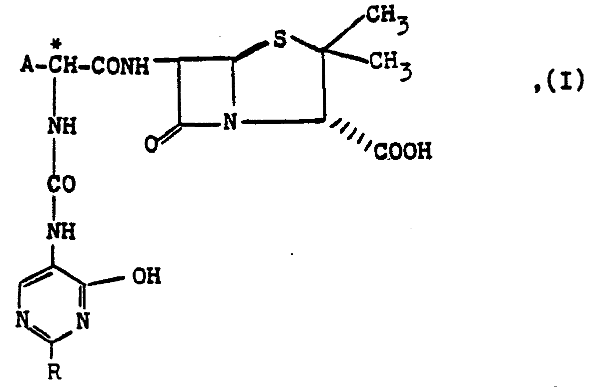 ihre physiologisch verträglichen Salze mit anorganischen oder organischen Basen, Verfahren zur Herstellung dieser Verbindungen und diese Verbindungen enthaltende Arzneimittel.

In der obigen allgemeinen Formel I bedeutet:
a) A die Phenylgruppe und R die Gruppe der allgemeinen Formel II, 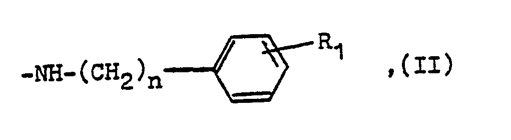 in der n=O oder 1 ist und R₁, sofern n=0 ist, die Gruppen 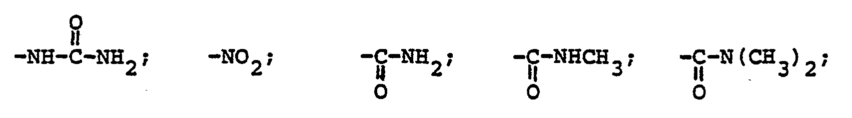 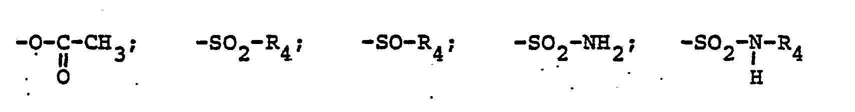 oder -SO₂-N(R₄)₂ bedeutet und, sofern n=1 ist, bedeutet es die obengenannten Gruppen und noch zusätzlich ein Wasserstoff-'oder Fluoratom, die Hydroxy-, Methyl- oder Methoxygruppe, wobei R₄ in den obigen Formeln eine Alkylgruppe mit 1 bis 4 Kohlenstoffatomen bedeutet, oder R eine Gruppe der allgemeinen Formel III, 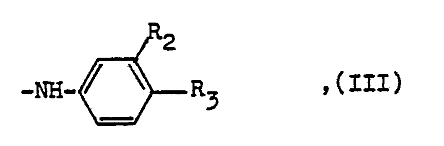 in der einer der Reste R₂ oder R₃ die Hydroxygruppe bedeutet und der andere dieser Reste eine Gruppe der Formeln 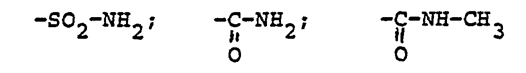 oder die beiden Reste R₂ und R₃ die Gruppe -CONH₂ darstellen,1 oder es bedeutet b) A die p-Hydroxyphenylgruppe und R die Gruppe der allgemeinen Formel II, in der n=0 oder 1 ist und R₁, sofern n=0 ist, die Gruppen der Formeln 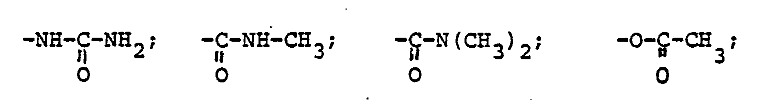 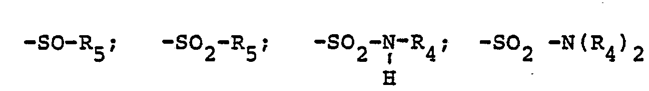 oder die meta-ständige Gruppe -S0₂-NH₂ bedeutet, und, sofern n=1 ist, bedeutet es die obengenannten Gruppen und noch zusätzlich ein Fluoratom, die Hydroxygruppe, die Nitrogruppe, die Gruppe H₂NCO-, die Gruppen -SO-CH₃, -SO₂-CH_{3'} .oder p-H₂N-SO₂-, oder die Methyl- oder Methoxygruppe wobei in den obigen Formeln R₄ wie weiter oben angegeben definiert ist und R₅ eine Alkylgruppe mit 2 bis 4 Kohlenstoffatomen bedeutet, oder R eine Gruppe der allgemeinen Formel III, in der einer der Reste R₂ oder R₃ die Hydroxygruppe bedeutet und der andere dieser Reste eine Gruppe der Formeln 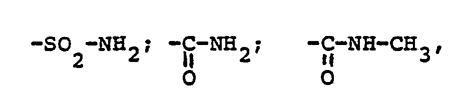 oder in der die beiden Reste R₂ und R₃ die Gruppe -CONH₂ darstellen, oder es bedeutet
c) A die 2-Thienylgruppe, die Cyclohexa-1,4-dien-1-ylgruppe, die 3,4-Dihydroxyphenyl- oder die 3-Chlor-4-hydroxyphenylgruppe und R eine Gruppe der allgemeinen Formel II, in der n=0 oder 1 ist und R₁ ein Fluoratom, die Methyl- oder Methoxygruppe oder eine Gruppe der Formeln 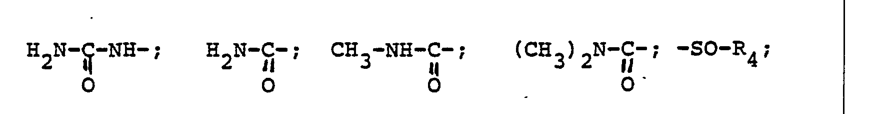 -SO₂-R₄: -SO₂-NH₂, 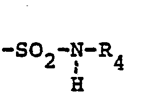 oder -SO₂-N(R₄)₂ darstellt, wobei in diesen Formeln R₄ wie oben angegeben definiert ist, und R₁ auch ein Wasserstoffatom sein kann, sofern dann A die 2-Thienyl-, 3,4-Dihydroxyphenyl- oder 3-Chlor-4-hydroxyphenylgruppe bedeutet oder R₁ die Hydroxygruppe oder die Methylcarbonyloxygruppe, sofern A die 2-Thienylgruppe, die Cyclohexa-1,4-dien-1-ylgruppe oder die 3-Chlor-4-hydroxyphenylgruppe darstellt, oder R eine Gruppe der allgemeinen Formel III, in der R₂ die Hydroxygruppe und R₃ die Hydroxygruppe oder die Gruppen -CONH₂, -CO-NHCH₃,-SO₂-NH₂ bedeuten, beide Reste R₂ und R₃ können auch gleichzeitig die Gruppe -CONH₂ bedeuten.

Bevorzugt sind diejenigen Verbindungen der allgemeinen Formel I, worin R die Gruppen der Formeln 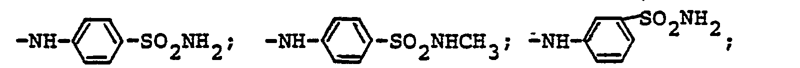 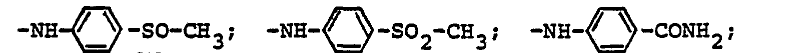 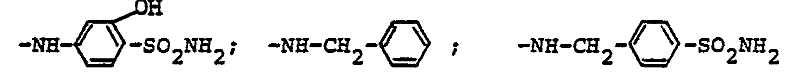 oder 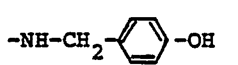 bedeuten. Als besonders wertvoll haben sich solche Verbindungen der allgemeinen Formel I erwiesen, bei denen R die Gruppe 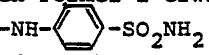 und A die Phenyl-, 2-Thienyl-, 3-Chlor-4-hydroxyphenyl- oder die 3,4-Dihydroxyphenylgruppe, oder R die Gruppe 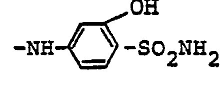 und A die Phenyl-, p-Hydroxyphenyl-, 2-Thienyl-, 3-Chlor-4-hydroxyphenyl- oder 3,4-Dihydroxyphenylgruppe oder R die Gruppe 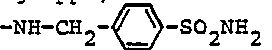 und A die Phenyl-oder p-Hydroxyphenylgruppe bedeutet.

Die Penicillinverbindungen der allgemeinen Formel I können in zwei tautomeren Formen (nämlich vom Lactim- und vom Lactamtyp) vorliegen. Es hängt besonders vom jeweiligen Lösemittel und von der Art des Substituenten R ab, welche der beiden Formeln I oder I' überwiegt:

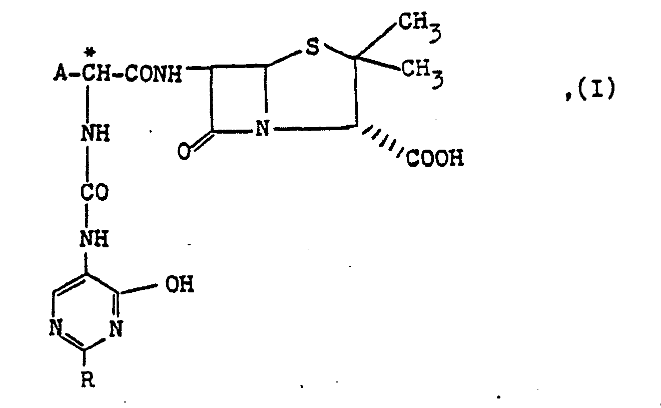

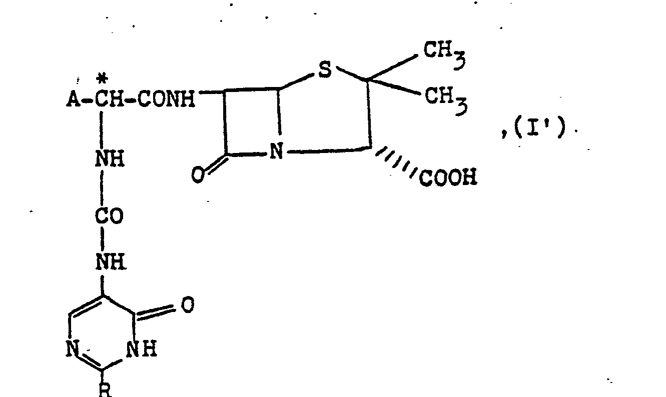

Es versteht sich von selbst, daß die eingangs angegebenen Verbindungen vom Typ I immer beide Tautomeren umfassen.

Die Verbindungen der allgemeinen Formel I können bezüglich des Chiralitätszentrums C+ in den beiden möglichen R- und S-Konfiguratipnen, jedoch auch als ein Gemisch dieser beiden Konfigurationen, vorliegen. Besonders bevorzugt sind solche Verbindungen, für welche die D=_{R}-Konfiguration zutrifft.

Die neuen halbsynthetischen Penicilline der allgemeinen Formel I zeigen ein breites Wirkungsspektrum gegen grampositive und gramnegative Bakterien bei einer sehr guten Verträglichkeit beim Menschen.

Bekanntlich inhibieren Penicillin-Antibiotika das Wachstum verschiedener grampositiver und gramnegativer Bakterien. Es ist ferner bekannt, daß nur wenige Penicilline gute Wirkung gegen wichtige gramnegative Problemkeime, die vor allem im Hospitalbereich auftreten, wie Pseudomonas und Klebsiella, besitzen. Während der letzten Jahre ist jedoch die Häufigkeit des Auftretens von Infektionen, die durch diese Keime, insbesondere durch Pseudomonas-Arten, hervorgerufen wurde, ständig gestiegen. Penicillin-Derivate wie Carbenicillin (US-Patentschrift 3 142 673), Sulbenicillin (US-Patentschrift 3 660 379) sowie Ticarcillin (US-Patentschrift 3 282 926) werden zwar als antipseudomonale Antibiotika beschrieben, weisen jedoch in vitro wie in vivo nur eine mäßige Wirksamkeit auf. Eine wichtige Weiterentwicklung sind acylierte Derivate von α-Aminobenzylpenicillinen, z.B. Ampicillin und Amoxycillin. Aus diesen, in den letzten Jahren intensiv bearbeiteten Verbindungsklassen wurde vor kurzem Azlocillin = 6-{D-α'-[(2-Oxo-imidazolidin-1-yl)-carbonylamino]-4-phenylacetamido}-penicillansäure-Natriumsalz (z.B. Belgische Patentschrift 767 647) als weiteres Antipseudomonas-Penicillin eingeführt. Für eine erfolgreiche Behandlung muß dieses Penicillin jedoch hoch dosiert werden. Außerdem ist seine Wirkung gegen Klebsiella und E.coli-Arten nur mäßig. Es bestand daher weiter ein Bedürfnis, nach neuen Penicillinen zu suchen, die eine gesteigerte Wirksamkeit gegen Bakterien, wie Pseudomonas bzw. Klebsiella und E.coli, besitzen.

Während wie erwähnt, allgemein über Acylderivate von a-Aminobenzylpenicillinen intensiv geforscht wurde und noch wird, ist nur wenig über Derivate bekannt geworden, bei denen ein Heterocyclus über eine Ureido-Brücke 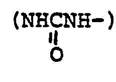 an das α-Benzylkohlen- stoffatom vono α-Aminobenzylpenicillinen geknüpft ist. Lediglich in den DE-OS 2 450 668 und 2 535 655 und der US-Patentschrift 4 031 230 werden Hydroxypyridylureido-benzylpenicilline der allgemeinen Formel IV beschrieben:

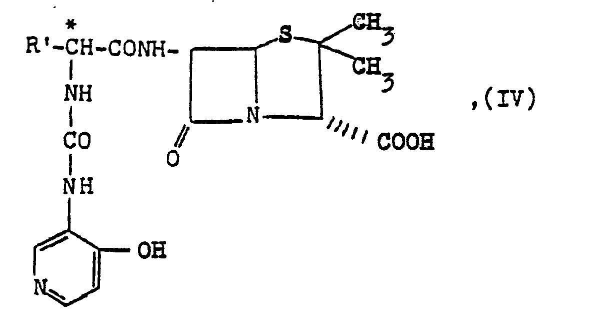

Diese Verbindungen sind jedoch in ihrer antibakteriellen Aktivität den Verbindungen der vorveröffentlichten europäischen Patentanmeldung Nr. 79 100 468.2, die sich von den erfindungsgemäßen Verbindungen nur durch andere Bedeutungen des Restes R oder durch andere Kombinationen der Reste A und R unterscheiden, deutlich unterlegen. Die erfindungsgemäßen Verbindungen besitzen aber im Vergleich zu den Verbindungen dieser europäischen Patent anmeldung gegenüber Problemkeimen, insbesondere gegen Bakterien, wie E.coli, Pseudomonas und Klebsiella, noch bessere Aktivitäten.

Die Verbindungen der allgemeinen Formel I lassen sich wie folgt darstellen:
1) Durch Umsetzung einer Verbindung der allgemeinen Formel V, 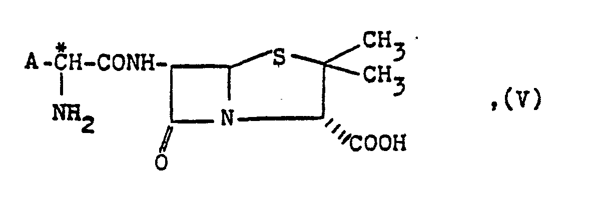 in der A wie oben definiert ist, mit einem Pyrimidinderivat der allgemeinen Formel VI, 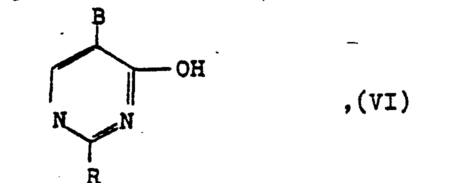 in der R wie oben definiert ist und B die Gruppe -NCO oder ein reaktives Derivat der Gruppe NHCOOH bedeutet, wie z.B. die Gruppen -NHCOC1, -NHCOBr oder 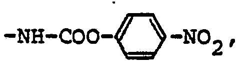 wobei die Gruppe NHCOCl besonders bevorzugt ist. Es können auch Gemische von solchen Pyrimidinderivaten der allgemeinen Formel VI verwendet werden, in der B teils die eine und teils die andere der vorstehend genannten Bedeutungen besitzt, z.B. die Gruppen -NCO und 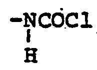 gleichzeitig nebeneinander.

Die Ausgangsprodukte der allgemeinen Formel V können in Form ihrer anorganischen oder organischen Salze, z.B. als Triäthyl ammoniumsalz oder Natriumsalz, eingesetzt werden. Die Reaktion kann dann in beliebigen Mischungen von Wasser mit solchen organischen Lösungsmitteln, die mit Wasser mischbar sind, wie Ketonen, z.B. Aceton, cyclische Äther, z.B. Tetrahydrofuran, oder Dioxan, Nitrilen z.B. Acetonitril, Formamiden, z.B. Dimethylformamid, Dimethylsulfoxid oder Alkoholen z.B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z.B. halogenierten Kohlenwasserstoffen, wie Chloroform oder Methylenchlorid unter Zusatz von Basen, vorzugsweise Triäthylamin, Diäthylamin oder N-Äthylpiperidin durchzuführen.

Weiterhin läßt sich die Reaktion in einem Gemenge aus Wasser und einem mit Wasser nicht mischbaren Lösungsmittel, wie Äther, z.B. Diäthyläther, halogenierten Kohlenwasserstoffen, z.B. Chloroform oder Methylenchlorid, Schwefelkohlenstoff, Ketonen, z.B. Isobutylmethylketon, Estern, z.B. Essigsäureäthylester, aromatischen Lösungsmitten, z.B. Benzol ausführen, wobei es zwe ckmäßig ist, kräftig zu rühren, und den pH-Wert durch Basenzusatz oder Verwendung von Pufferlösungen in einem Bereich von etwa pH 2,0 bis 9,0, vorzugsweise zwischen 6,5 und 8,0 zu halten. Man kann die Reaktion aber auch in Wasser allein in Gegenwart einer organischen oder anorganischen Base oder unter Zusatz von Pufferstoffen durchführen.

Verwendet man als Ausgangsprodukte für das erfindungsgemäße Verfahren die Silylderivate der Verbindungen der allgemeinen Formel V (z.B. Mono- oder Di-trimethylsilylderivate) und setzt sie mit Verbindungen der allgemeinen-Formel VI um, so arbeitet man im allgemeinen zweckmäßigerweise in wasser- und hydroxyl-gruppenfreien Lösungsmitteln, beispielsweise in halogenierten Kohlenwasserstoffen, z.B. Methylenchlorid oder Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmäßigerweise tertiäre aliphatische oder aromatische Amine, wie Pyridin oder Triäthylamin, oder durch sterische Hinderung schweracylierbare sekundäre Amine, wie Dicyclohexylamin, benützt.

Statt der Silylester können auch alle anderen Carboxylderivate vond-Aminobenzylpenicillinen, die auf dem Gebiet der Herstellung halbsynthetischer Penicilline bekannt sind, verwendet werden. Typische Beispiele sind die Tritylester, die p-Nitrobenzylester oder die Phenacylester. Im Anschluß an die Umsetzungen können diese Derivate nach bekannten Methoden in die erfindungsgemäßen Penicilline umgewandelt werden. Die Menge der verwendeten Basen ist z.B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der all- gemeinen Formel IV vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogen-. carbonate, Ammoniak, primäre, sekundäre und tertiäre aliphatische und aromatische Amine sowie heterocyclische Basen. Beispielhaft seien Natrium-, Kalium- und Calziumhydroxid, Calziumoxid, Natrium- und Kaliumcarbonat, Natrium- und Kaliumhydrogencarbonat, Äthylamin, Methyl-äthylamin, Triäthylamin, Hydroxyäthylamin, Anilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme können alle üblichen Puffermischungen verwendet werden, z.B. Phosphatpuffer, Citratpuffer und Tris(hydroxymethyl)amino-methan-Puffer.

Die Reaktionstemperaturen können in einem größeren Bereich variiert werden. Im allgemeinen arbeitet man zwischen etwa -20 und etwa +50°C, vorzugsweise zwischen 0 und +20°C.

Die Reaktionspartner der allgemeinen Formeln IV und V können von vornherein in äquimolaren Mengen miteinander zur Reaktion gebracht werden. Es kann aber in einzelnen Fällen durchaus zweckmäßig sein, einen der beiden Reaktionspartner im Überschuß zu verwenden, um sich damit die Reinigung des Endproduktes zu erleichtern oder um die Ausbeute zu steigern.

2) Durch Umsetzung von Ureidocarbonsäuren der allgemeinen Formel VII, 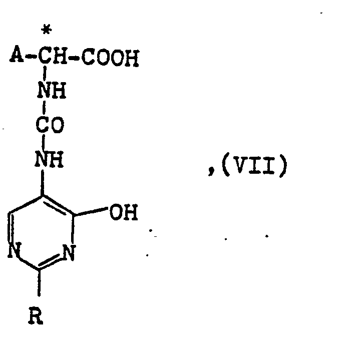 in der A und R die oben angegebenen Bedeutungen besitzen, ihren Salzen oder reaktiven Derivaten, mit der 6-Aminopenicillansäure der Formel VIII, 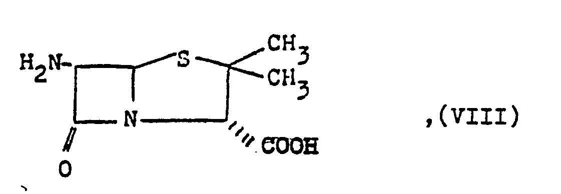 oder ihren anorganischen oder organischen Salzen oder Derivaten, die in die 6-Aminopenicillansäure leicht überführbar sind. Das dabei entstehende Reaktionsprodukt wird gegebenenfalls anschließend zu einem Penicillin der allgemeinen Formel I hydrolysiert oder katalytisch hydrogenolisiert.

Als reaktive Derivate der Ureidocarbonsäuren der allgemeinen Formel VII kommen beispielsweise deren Säureanhydride wie z.B die, die sich von Chlorameisenäureestern, z.B. Chlorameisensäureäthyl- oder -isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage. Prinzipiell können jedoch alle Verknüpfungsmethoden, wie sie aus der β-Lactamchemie bekannt sind, verwendet werden.

Die 6-Aminopenicillansäure wird vorteilhafterweise in Form eines ihrer Derivate eingesetzt. Als Derivate kommen hierfür z.B. in Frage: ihr Trimethylsilylester, Tritylester, p-Nitrobenzylester, Phenacylester und ihr O,N-Bis-trimethyl- silylderivat. Diese Derivate werden bevorzugt in einem aprotischen Lösungsmittel, wie Methylenchlorid oder Tetrahydrofuran, umgesetzt. Man kann aber auch die'6-Aminopenicillansäure in Form ihrer Salze, beispielsweise ihres Triäthylammoniumsalzes, zum Einsatz bringen; hierbei benutzt man z.B. Methylenchlorid oder ein protisches Lösungsmittel oder ein wäßriges Medium oder ein wäßrig-organisches Lösungsmittel, wie z.B. Tetrahydrofuran-Wasser-Gemische.

Die Ureidocarbonsäure, ihre Salze oder ihre reaktiven Derivate werden mit der 6-Aminopenicillansäure oder ihren Derivaten in einem Lösungsmittel bei Temperaturen zwischen -40°C und +40°C, gegebenenfalls in Gegenwart einer Base, umgesetzt. Wird z.B. ein Anhydrid der Ureidocarbonsäure, beispielsweise das Anhydrid mit dem Äthylchloroformiat, mit einem Derivat der 6-Aminopenicillansäure umgesetzt, so wird die Reaktion unter Kühlung, beispielsweise bei -10°C bis +10°C in Gegenwart eines tertiären Amins, wie Triäthylamin oder N,N-Dimethylanilin, in einem Lösungsmittel, wie Aceton, Tetrahydrofuran, Dimethylformamid, Chloroform,

Dichlormethan, Hexametapol oder in einem Gemisch dieser Lösungsmittel, durchgeführt. Setzt man beispielsweise einen N-Hydroxysuccinimidester der Ureidocarbonsäure mit der 6-Aminopenicillansäure um, so wird die Reaktion vorzugsweise bei 0 bis 20°C in Anwesenheit einer Base, wie z.B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel VII selbst oder ihrer Salze mit der 6-Aminopenicillansäure oder mit deren Salzen erfolgt vorteilhafterweise in Gegenwart eines Kondensationsmittels, z.B. in Gegenwart von N,N'-Dicyclohexylcarbodiimid.

Wird ein Derivat der 6-Aminopenicillansäure eingesetzt, beispielsweise einer ihrer obengenannten Ester, so wird je nach den Reaktionshedingungen gegebenenfalls ein Reaktionsprodukt erhalten, welches z.B. noch die Esterfunktion enthält. Ein solches Reaktionsprodukt ist aber leicht in das Penicillin der allgemeinen Formel I überführbar. Liegt zum Beispiel die Carboxylgruppe der 6-Aminopenicillansäure in Form ihres Silylesters vor, so kann sie nach der Reaktion in dem erhaltenen Penicillin der allgemeinen Formel I ebenfalls in Form ihres Silylesters vorliegen. In diesem Fall wird anschließend an die eigentliche Umsetzung diese Silylestergruppe abhydrolysiert, wodurch die Verbindung der allgemeinen Formel I entsteht. In anderen Fällen, z.B. bei Vorliegen eines p-Nitrobenzylesters, wird nach der eigentlichen Umsetzung diese p-Nitrobenzylestergruppe hydrogenolytisch gespalten, wobei dann das Penicillin der allgemeinen Formel I erhalten wird.

Die Aufarbeitung des nach beiden Verfahren erhaltenen Reaktionsgemisches nach erfolgter Umsetzung wird nach den bei B-Lactam-Antibiotika gebräuchlichen Methoden vorgenommen; dasselbe gilt für die Isolierung und Reinigung der Endprodukte, beispielsweise für die Freisetzung der Säure aus ihren Salzen und die Überführung der freien Säure in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanoat.

Die als Ausgangsstoffe verwendeten Verbindungen der allgemeinen Formel V sind literaturbekannt, vgl. z.B. E.H. Flynn, Cephalosporines and Penicillines, Academic Press, New York and London (1972).

Die Ausgangsstoffe der allgemeinen Formel VI können beispielsweise durch Umsetzung der entsprechenden 5-Aminopyrimidine der allgemeinen Formel IX, 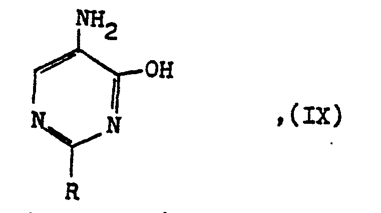 in der R wie oben definiert ist, mit Phosgen gewonnen werden. Diese Umsetzung erfolgt vorzugsweise in einem nicht Hydroxylgruppen-enthaltenden Lösungsmittel, wie Tetrahydrofuran, Methylenchlorid, Chloroform, Dimethoxyäthan oder Hexametapol bei Temperaturen zwischen -40° und +60°C, vorzugsweise zwischen -10⁰ und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuß verwendet werden. Sollten die betreffenden Aminopyrimidine der allgemeinen Formel IX in einem der erwähnten Lösungsmittel schwer löslich sein, kann die Phosgenierung auch in heterogener Phase durchgeführt werden. Des weiteren können die Aminopyrimidine der allgemeinen Formel IX durch Behandlung mit einem Silylierungsmittel, wie Hexamethyldisilazan oder Trimethylchlorsilan/Triäthylamin oder N,O-Bistrimethylsilyl- acetamid, in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel VI reagiert. Je nach der Art des Lösungsmittels, der Höhe der Temperatur, der Menge und Art der eingesetzten Base entsteht entweder überwiegend das entsprechende Isocyanat oder Carbaminsäurehalogenid oder ein Gemisch dieser beiden Verbindungen. Je nach den Reaktionsbedingungen kann die Verbindung der allgemeinen Farmel VI auch gering oder teilweise als ein, den Isocyanaten isomeres Dihydro-öxazolo-pyrimidin der allgemeinen Formel VIa 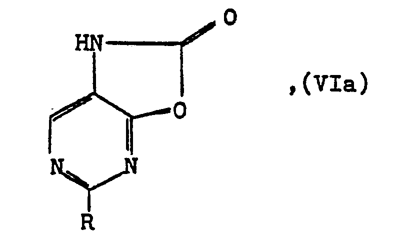 oder bei vorhergehender Silylierung je nach Art des Substituenten R als ein ein- oder mehrfach silyliertes Analoges vorliegen.

Die durch Phosgenierung entstandenen Ausgangsprodukte der allgemeinen Formel VI bzw. deren Gemische sind in den obeneriwähnten Lösungsmitteln im allgemeinen gut löslich und können, nach Entfernung des überschüssigen Phosgens, ohne weitere Reinigung mit den entsprechenden Penicillinderivaten der allge- meinen Formel V direkt umgesetzt werden. Es ist jedoch auch möglich, das Zwischenprodukt der allgemeinen Formel VIa zu isolieren, es gegebenenfalls mit einem protischen Lösungsmittel, beispielsweise Wasser oder Methanol, zu entsilylieren, es auf Grund seiner Löslichkeitseigenschaften zu reinigen und in der oben dargelegten Weise umzusetzen.

Die Darstellung der 2-substituierten 5-Amino-4-hydroxy- pyrimidine der allgemeinen Formel IX wird, soweit sie nicht in der offengelegten Europäischen Patentanmeldung Nr. 79 100 468.2 bereits beschrieben sind, in den nachfolgenden Beispielen genauer angegeben. Besonders gut eignet sich die Umsetzung von 2-Chlor-4-hydroxy-5-nitro-pyrimidin mit Anilinen oder Benzylaminen in wäßriger Lösung, wobei anschließend die Nitrogruppe nach an sich bekannten Methoden reduziert wird, oder die Umsetzung von 5-Benzoylamino-4-hydroxy-2-methylmercapto-pyrimidin mit den entsprechenden Anilinen oder Benzylaminen in der Schmelze oder in einem hochsiedenden Lösungsmittel, wie z.B. Sulfolan.

Di Ureidocarbonsäuren der allgemeinen Formel VII lassen sich leicht durch Umsetzung der Pyrimidinderivate der allgemeinen Formel VI mit Glycinderivaten der allgemeinen Formel X, 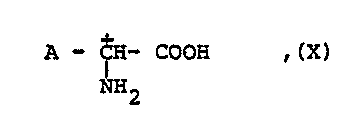 in der A wie oben definiert ist, erhalten. Die Umsetzung erfolgt bei Temperaturen zwischen -20° und +40°C, vorzugsweise zwischen 0° und +20°, in einem Lösungsmittel. Als Lösungsmittel können hierbei z.B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin oder anorganische Basen, wie verdünnte Natronlauge.

Es wurde gefunden, daß die Verbindungen der allgemeinen Formel I wertvolle pharmakologische Eigenschaften bei guter Verträglichkeit besitzen. Die erfindungsgemäßen Wirkstoffe können daher zur Prophylaxe und Chemotherapie von lokalen und systemischen Infektionen in der-Human- und Tiermedizin verwendet werden. Als Krankheiten, die durch die erfindungsgemäße Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis'und allgemeine systemische Infektionen. Weiter können diese Verbindungen als Stoffe zur Konservierung von anorganischen oder organischen Materialien verwendet werden, besonders von organischen Materialien, wie Polymeren, Schmiermittel, Farben, Fasern, Leder, Papier und Holz sowie von Lebensmitteln.

Dieses wird dadurch ermöglicht, daß die Verbindungen der allgemeinen Formel I sowohl in vitro als auch in vivo gegen schädliche Mikroorganismen, insbesondere gegen grampositive und gramnegative Bakterien und bakterienähnliche Mikroorganismen sehr stark wirken, wobei sie sich besonders durch ein breites Wirkungsspektrum auszeichnen. Daneben zeigen die Verbindungen der allgemeinen Formel I nach parenteraler Gabe hohe Spiegel in Gewebe, Serum, Organen und im Urin.

In der folgenden Tabelle 1 werden typische, besonders gut wirksame Penicilline gemäß vorliegender Erfindung aufgezählt. Die genannten Penicilline können nach dem Verfahren 1 oder 2 hergestellt werden:

Es handelt sich um Verbindungen der allgemeinen Formel I, in der A und R wie folgt definiert sind:

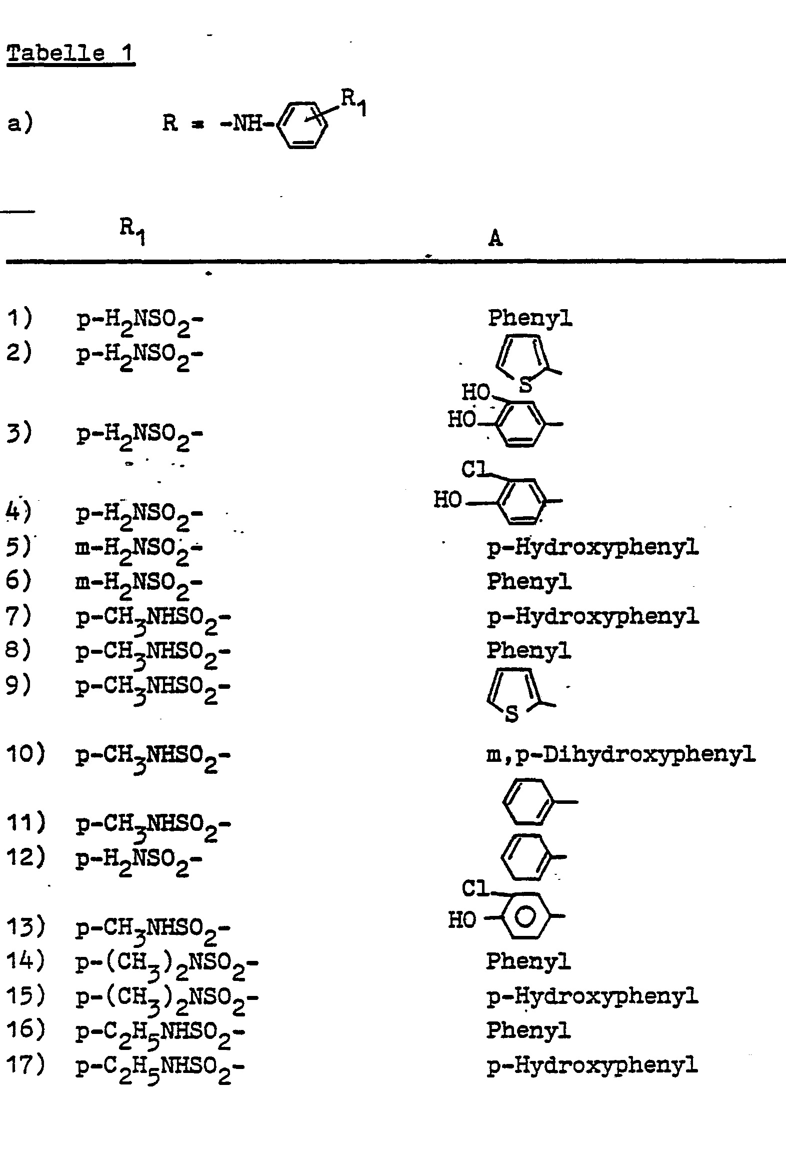

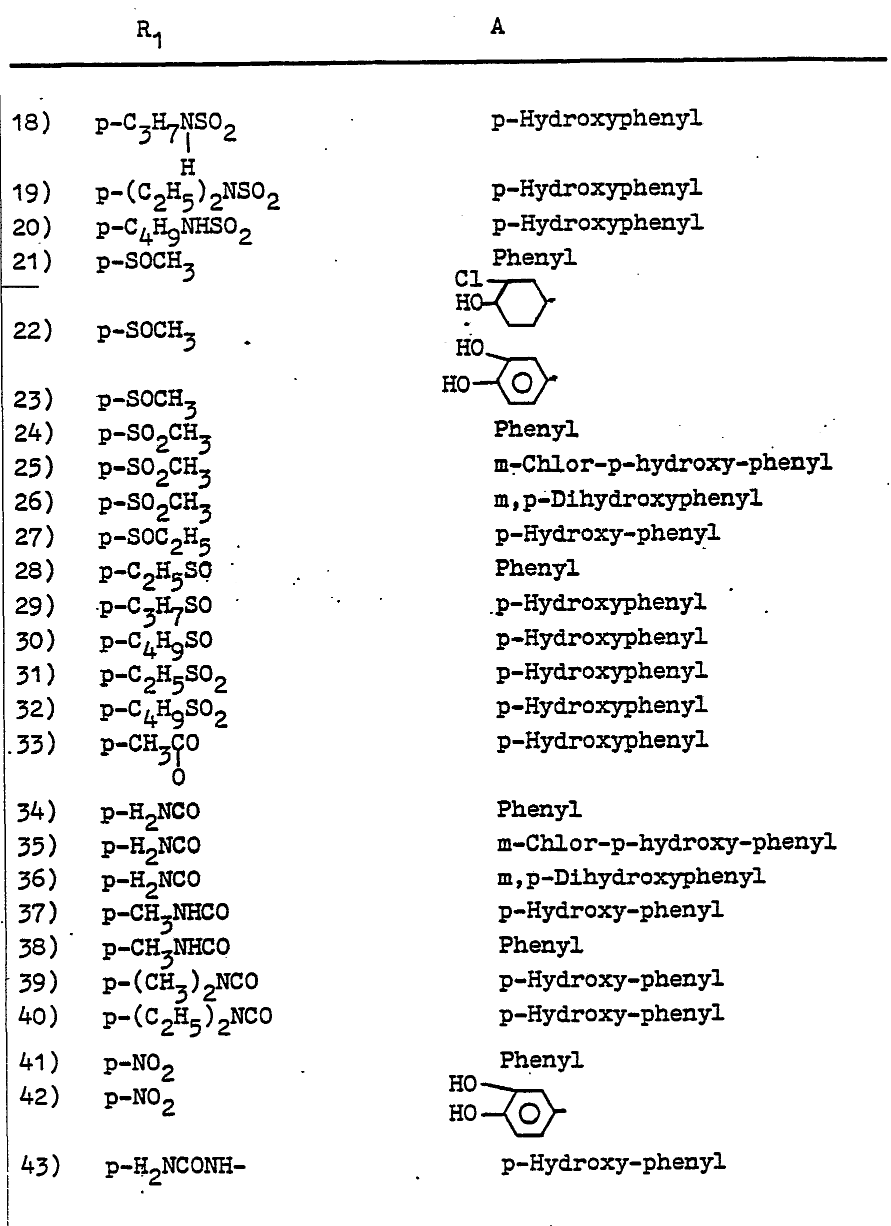

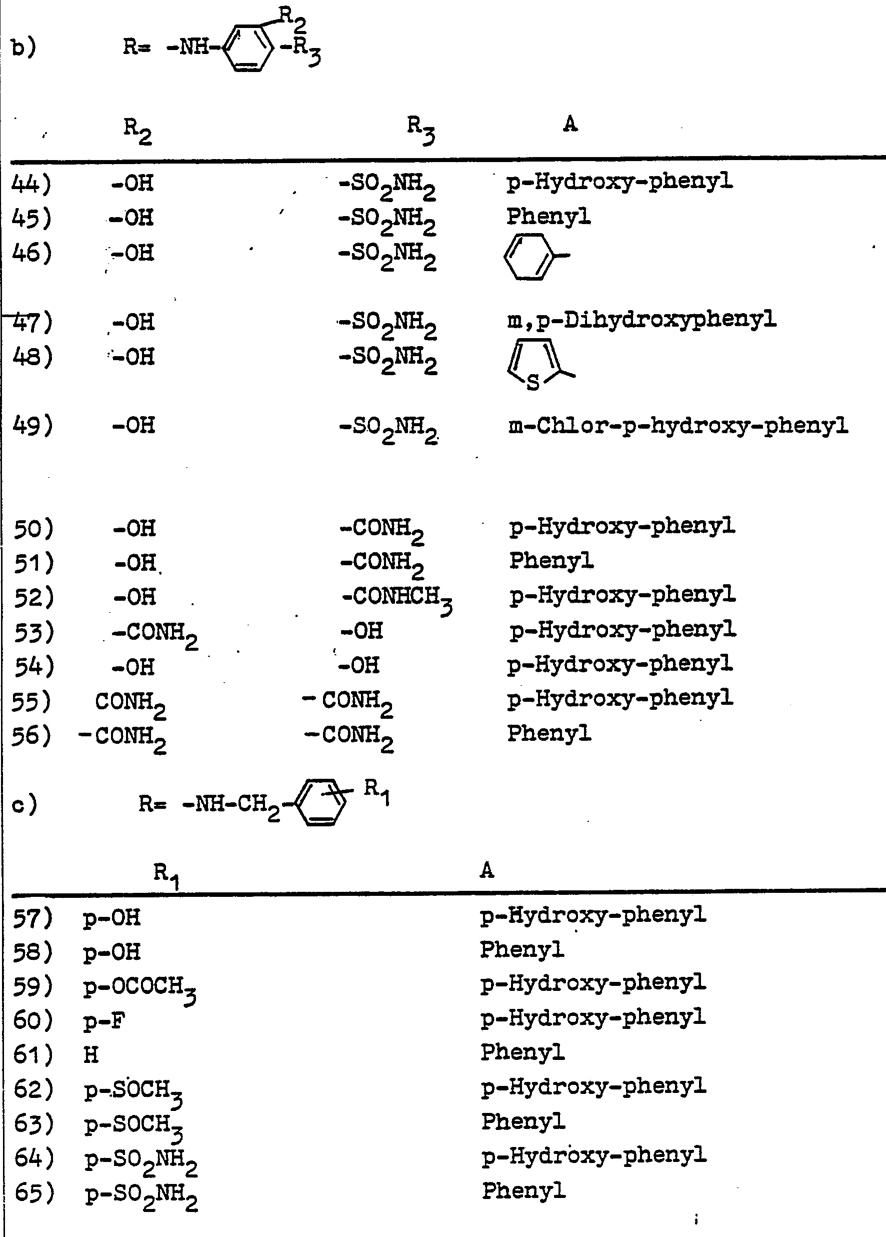

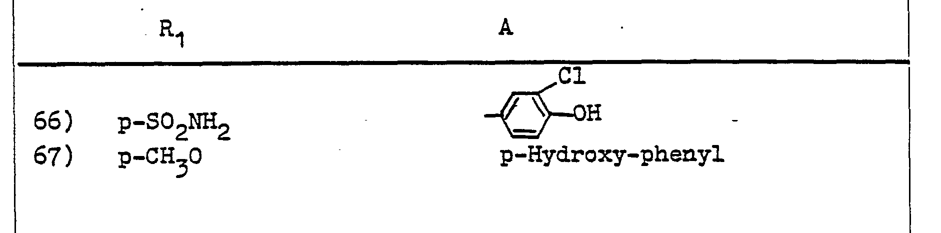

Mit diesen Penicillinderivaten können beispielsweise lokale und/oder systemische Erkrankungen behandelt und/oder verhindert werden, die durch die folgenden Erreger oder durch Mischungen der folgenden Erreger verursacht werden:
Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe, Klebsiella-Bakterien, z.B. K. pneumoniae;
Proteae-Bakterien der Proteus-Gruppe z.B. Proteus vulgaris;
Salmonella-Bakterien, z.B. S.thyphimurium;
Shigella-Bakterien, z.B. Shigella dysenteriae;
Pseudomonas-Bakterien, z.B. Pseudomonas aeruginosa;
Aeromonas-Bakterien, z.B. Aeromonas lique faciens.
Spirillaceae, wie Vibrio-Bakterien, z.B. Vibrio cholerae;
Parvobacteriaceae oder Brucellaceae, wie Pasteurella-Bakterien;
Brucella-Bakterien, z.B. Brucella abortus;
Haemophilus-Bakterien, z.B. Haemophilus influenzae;
Bordatella-Bakterien, z.B. Bordetella pertussis;
Moraxella-Bakterien, z.B. Moraxella lacunata;
Bacteroidaceae, wie Bacteroides-Bakterien;
Fusiforme-Bakterien, z.B. Fusobacterium fusiforme;
Sphaerophorus-Bakterien, z.B. Sphaerophorus necrophorus;
Bacillaceae, wie aerobe Sporenbildner, z.B. Bacillus anthracis;
anaerobe Sporenbildner-Chlostridien, z.B. Chlostridium perfringens;
Spirochaetaceae, wie Borrelia-Bakterien;
Treponema-Bakterien, z.B. Treponema pallidum;
Leptospira-Bakterien, wie Leptospira interrogans.

Die obige Aufzählung von Erregern ist lediglich beispielhaft und keineswegs beschränkend aufzufassen.

Die Wirksamkeit der erfindungsgemäßen Penicilline läßt sich durch folgende Untersuchungen beispielhaft demonstrieren:

### 1. In vitro-Versuche

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht.

>.64; 64; 32; 16; 8; 4; 2; 1; 0,5; 0,25; 0,125; 0,06; 0,03; 0,015 und 0,007jug/ml.

Es wurde ein Nährboden folgender Zusammensetzung benutzt: 10 g Pepton, 8 g Fleischextrakt-Oxoid, 3 g Natriumschlorid, 2 g sek. Natriumphosphat werden mit dest. Wasser auf 1000 ml aufgefüllt (pH 7,2-7,4). Lediglich bei der Testung gegen Streptokokken wurde 1% Glucose hinzugefügt. Das Alter der Primärkulturen betrug etwa 20 Stunden. Die Einstellung der Keimsuspension erfolgte am Photometer (nach "Eppendorf") (Reagenzglas-Durchmesser 14 mm, Filter 546 nm) anhand der Trübung einer Bariumsulfat-Vergleichssuspension, die durch eine Bariumsulfat-Aufschwemmung erzeugt wurde, welche durch die Zugabe von 3,0 ml 1%ige Bariumchloridlösung in 97 ml 1%ige Schwefelsäure entstand. Nach der Einstellung wurden Streptococcus Aronson im Verhältnis 1:15 und die übrigen Testkeime im Verhältnis 1:1500 mit einer Kochsalzlösung weiter verdünnt.

16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenkonzentration (= niedrigste noch bakteriostatisch wirksame Konzentration) ermittelt wurde.

Als Testorganismen wurden benützt: - Staphylococcus aureus SG 511, Escherichia coli ATCC 11 775, Pseudomonas aeruginosa Hamburgensis und Walter, Serratia marcescens ATCC 13 880, Klebsiella pneumoniae ATCC 10 031 und BC 6, Proteus mirabilis Hamburgensis, Proteus rettgeri BC 7 und Enterobacter cloacae ATCC 13 047.

In der folgenden Tabelle 2 sind die ermittelten minimalen Hemmkonzentrationen (MIC) für typische Vertreter der erfindungsgemäßen Verbindungen aufgeführt, wobei sich die Ziffern auf die Penicilline der Tabelle 1 Seite 18 beziehen.

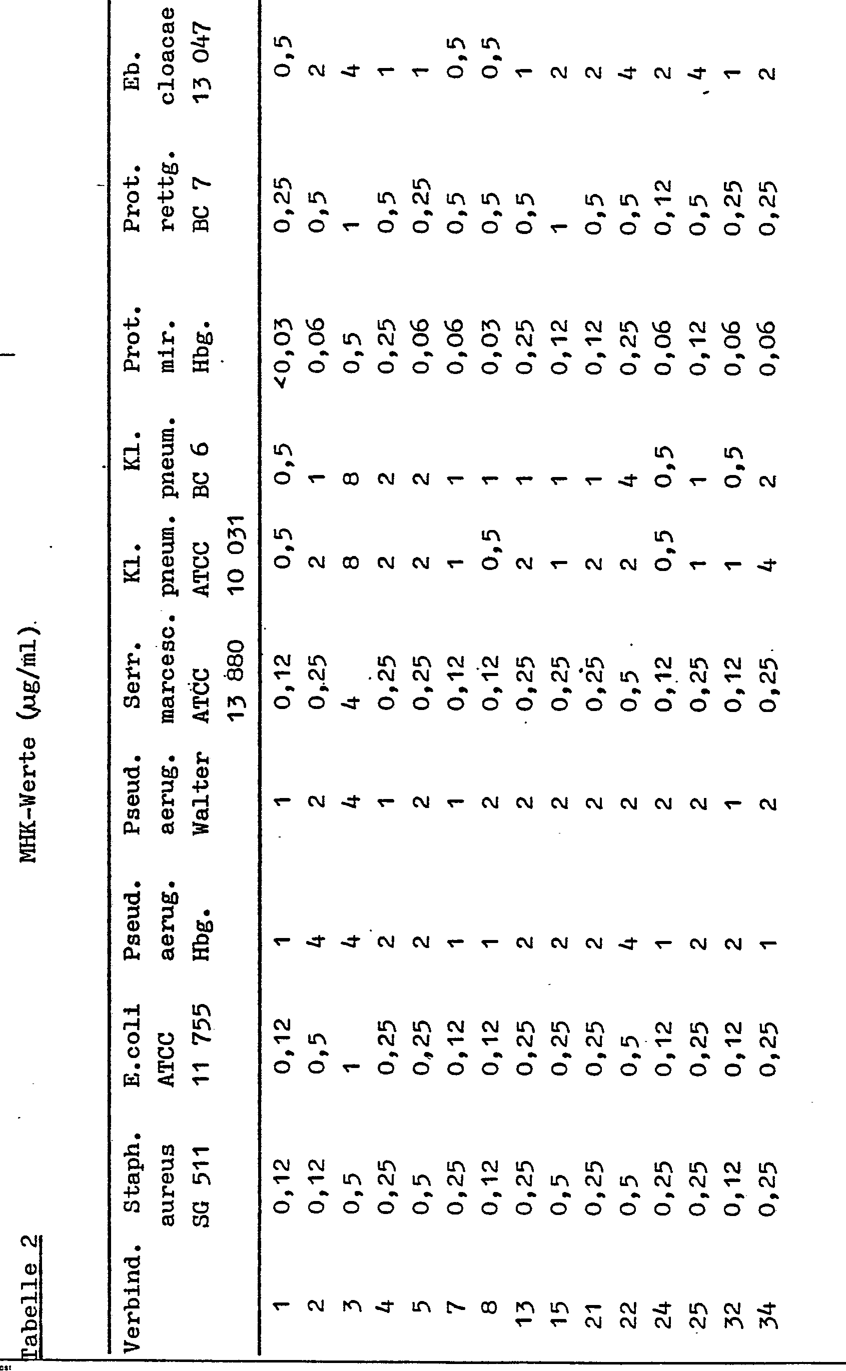

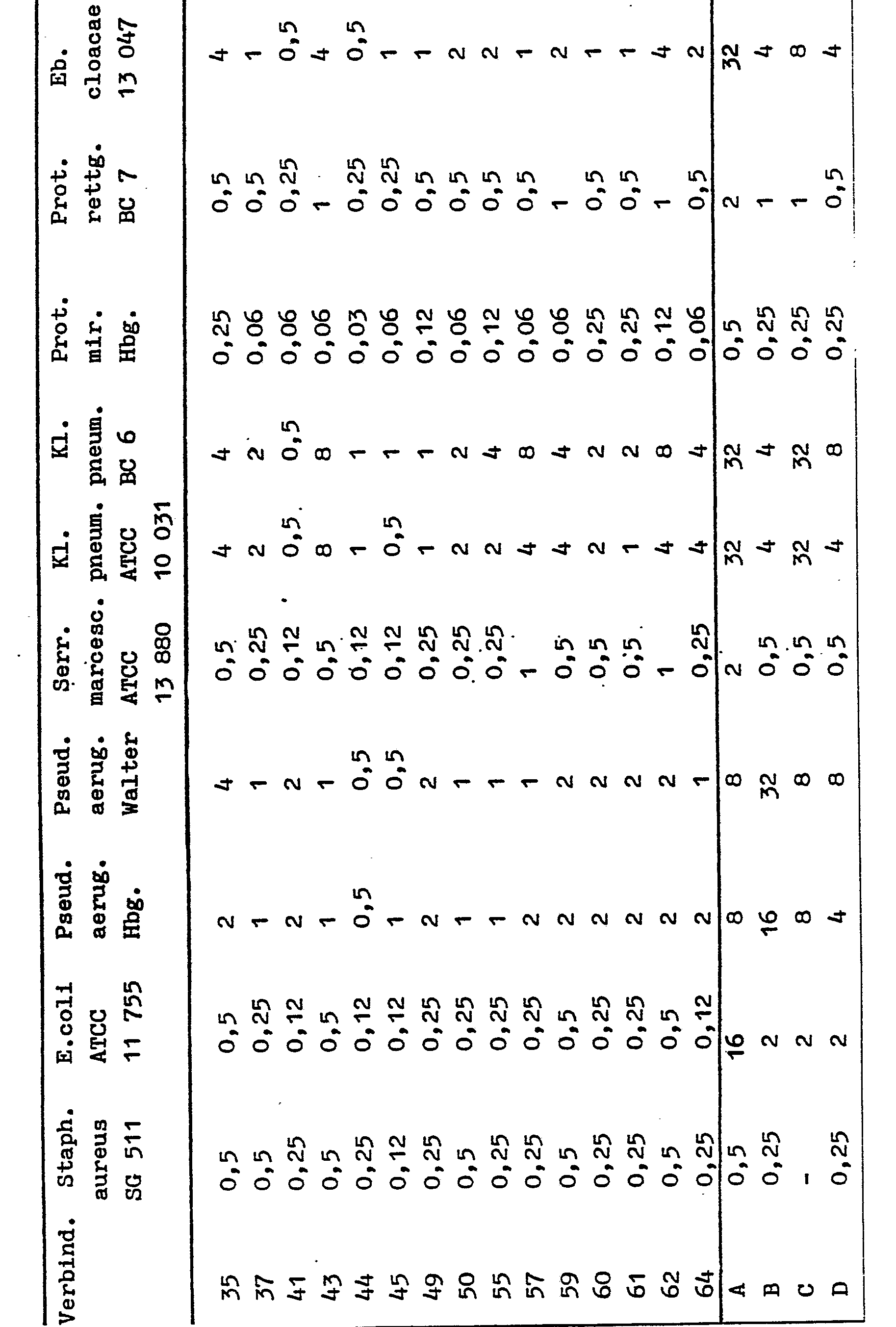

Eine Reihe der erfindungsgemäßen Verbindungen wurde in vivo bei experimentellen Infektionen an Mäusen untersucht. Man verwendete als pathogene Bakterien E.coli ATCC 11 775 und Pseudomonas aeruginosa Walter. Es wurde eine intraperitoneale Infektion mit 0,2 ml einer 5%igen Mucinsuspension der Bakterien gesetzt. Dies entspricht etwa 2x10⁶ Keime E.coli bzw. 8x10⁵ Keime Pseudomonas/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Penicilline zur Bestimmung der ED₅₀ (Dosis, bei der 50 % der Tiere überleben) behandelt. Bei der E.coli-Infektion wurde einmal therapiert. Bei der Pseudomonas-Infektion wurde dreimal behandelt.

Der Beobachtungszeitraum betrug in beiden Fällen 7 Tage. Die Ergebnisse dieser Teste mit repräsentativen Vertretern der erfindungsgemäßen Penicilline sind in der Tabelle 3 dargestellt.

Als Vergleichssubstanzen dienten die bekannten Penicilline der allgemeinen Formel

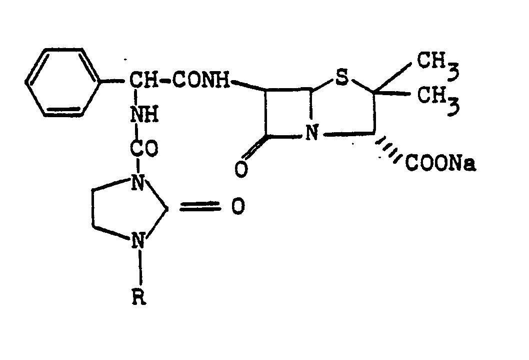

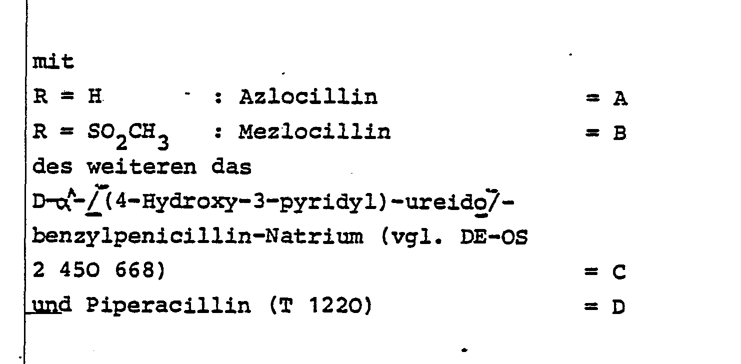

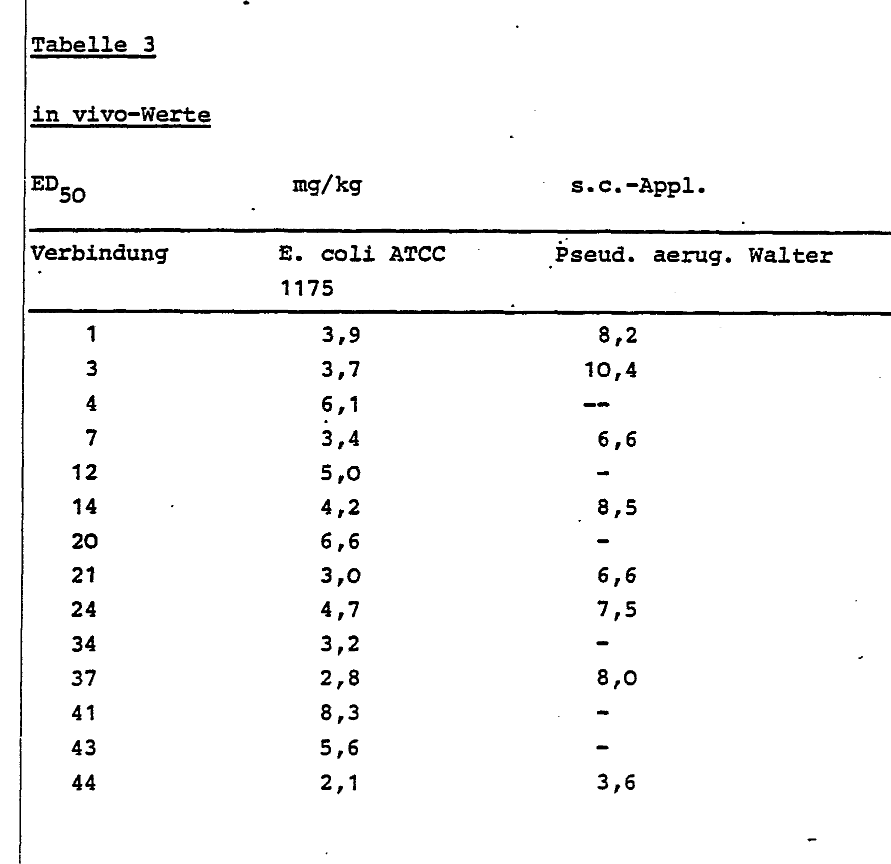

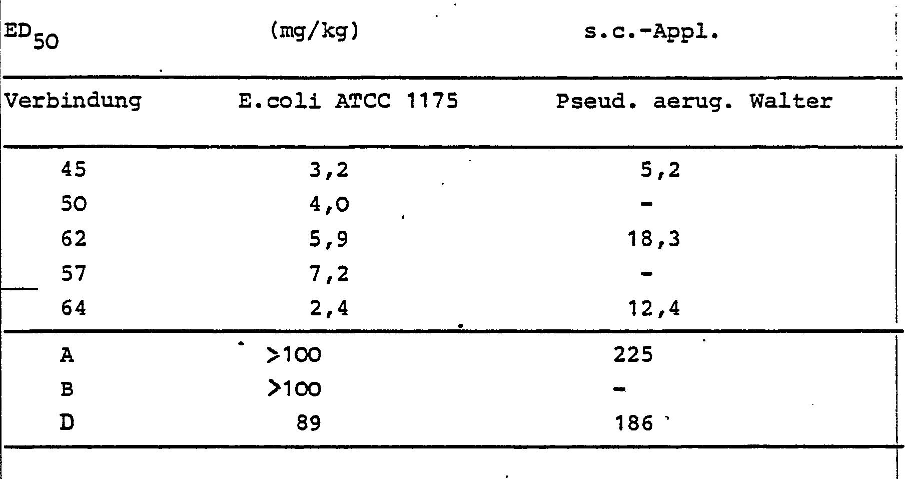

Die akute Toxizität wurde durch perorale und subcutane Applikation der Verbindungen der Tabelle 1 an weiße Laboratoriumsmäuse in steigenden Dosen bestimmt.

Die LD₅₀ ist die Dosis, nach deren Applikation 50 % der Tiere innerhalb von 8 Tagen sterben. Sämtliche Substanzen zeigen bei oraler Gabe eine LD₅₀ von über 4 g/kg, bei subcutaner Gabe eine LD₅₀ von über 3 g/kg, d.h. bei 3 g/kg starben keine Tiere, die Substanzen sind damit für die Praxis untoxisch.

Die angeführten Werte zeigen, daß repräsentative Vertreter der erfindungsgemäßen Penicilline auf Grund ihres breiten antibiotischen Spektrums, ihrer hohen antibakteriellen Aktivität, ihrer niederen Toxizität und ihres hohen Serumgehalts nach subcutaner und oraler Gabe wertvolle Antibiotika sind.

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragees, Kapseln, Granulate, Suppositorien, Lösungen, Suspensionen, Emulsionen, Salben, Gele, Cremes, Puder und Sprays genannt. Vorteilhafterweise wird in der Human- oder Tiermedizin der Wirkstoff oder ein Gemisch der verschiedenen Wirkstoffe der allgemeinen Formel I in einer Dosierung zwischen 5 und 500, vorzugsweise 10 - 200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 100, insbesondere 5 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels wowie dem Zeitraum bzw. Intervall, innerhalb welchem die Verabreichung erfolgt. So kann es in einigen Fällen ausreichend sein, t weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Bei der parenteralen Verabreichung wird es bevorzugt, die erfindungsgemäße Penicillinverbindung in einem nicht-toxischen flüssigen Medium für Injektionszwecke aufzulösen und die Lösung intramuskulär, intravenös oder subkutan zu injizieren.

Es ist auch möglich, die erfindungsgemäße Penicillinverbindung in einem nicht-toxischen flüssigen Medium oder in einer Salbengrundlage aufzulösen oder damit zu vermischen, und die Lösung oder das Gemisch direkt auf den geschädigten Ort aufzubringen. Die Verbindungen können auch als Suppositorien nach Vermischen mit einer Grundlage für Suppositorien oder nach dem Auflösen darin verwendet werden. - Beispiele für nicht-toxische flüssige Medien, die zur Herstellung von injizierbaren Zubereitungen, welche die Penicillinverbindung als Wirkstoff enthalten, verwendet werden können, sind sterilisiertes entionisiertes Wasser, physiologische Kochsalzlösung, Glucoselösung zur Injektion, Ringer'sche Lösung und Aminosäurelösung zur Injektion.

Die Penicillinverbindung kann auch in anderen injizierbaren Zubereitungen aufgelöst und parenteral verabreicht werden.

Für die orale Verabreichung werden die erfindungsgemäßen Penicillinverbindungen in Form eines pharmazeutischen Präparats verwendet, welches die Verbindung(en), gegebenenfalls in Mischung mit pharmazeutisch verträglichen Trägern, wie z.B. einem organischen oder anorganischen festen oder flüssigen Hilfsstoff, der sich für die orale Verabreichung eignet, enthält. Gewünschtenfalls können diese Präparate auch.Hilfs- substanzen, Stabilisatoren und andere üblicherweise verwendete Zusätze enthalten.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die folgenden Beispiele sollen die Erfindung näher erläutern: (Die Angabe der Rf-Werte bezieht sich auf das System n-Butanol- Wasser-Eisessig = 60:25:15, SiO₂-Platte). Mit "_{A}mpicillin" ist dasjenige α'-Aminobenzylpenicillin, mit "_{A}moxycillin" dasjenige α-Amino-p-hydroxy-benzylpenicillin und mit "Epicillin" dasjenige α'-Amino-α-(1,4-cyclohexadien-1-yl)-methylpenicillin mit der D=R-Konfiguration in der Seitenkette gemeint.

### I. Darstellung der Ausgangsverbindungen

### Beispiel A

### 5-Amino-4-hydroxy-2-p-methylsulfonylanilino-rimidin

3,2 g (0,019 Mol) 4-Methylthio-nitrobenzol werden in 20 ml eisensäure gelöst und bei Raumtemperatur 0,05 Mol Wasserstoffperoxid als 30%ige Lösung zugesetzt. Die Lösung erwärmt sich dabei. Nach 1 Stunde Stehen bei Raumtemperatur wird mit iswasser gefällt, der Niederschlag abgesaugt und bei 50°C im Vakuum getrocknet. Man erhält 2,9 g (76,2 % der Theorie) p-Nitrophenyl-methyl-sulfon.

3,42 g (0,017 Mol) p-Nitrophenyl-methyl-sulfon werden in 100 ml ethanol gelöst und in Gegenwart von 1 g Raney-Nickel bei 50°C und 5 bar hydriert. Die Lösung wird vom Katalysator abfiltriert und zur Trockne eingedampft. Der Rückstand wird in 40 ml Dioxan gelöst und eine Lösung von 3,77 g (0,0175 Mol) 2-Chlor-4-hydroxy-5-nitro-pyrimidin-Natriumsalzx 1H₂O in 120 ml Wasser zugegeben. Die Lösung wird 3 Stunden auf dem Dampfbad erhitzt. Nach dem Abkühlen wird der ausgefallene Nederschlag abgesaugt, mit Wasser gewaschen und bei 50°C im Vakuum getrocknet.

Ausbeute: 3,1 g (58,7 % der Theorie) 4-Hydroxy-2-p-methyl- sulfonylanilino-5-nitropyrimidin.

3,3 g (0,01063 Mol) 4-Hydroxy-2-p-methylsulfonylanilino-5-nitropyrimidin werden in 50 ml Wasser durch Zusatz von 6 ml konzentriertem Ammoniak und Erwärmen in Lösung gebracht. Unter Rühren werden 7,83 g (0,045 Mol) Natriumdithionit auf einmal zugesetzt. Anschließend wird noch 15 Minuten bei 60°C gerührt. Nach dem Abkühlen wird der entstandene Niederschlag abgesaugt, mit Wasser gewaschen und bei 50° C im Vakuum getrocknet.

Ausbeute: 2,4 g (80,6 % der Theorie) 5-Amino-4-hydroxy-2-p-methylsulfonylanilino-pyrimidin. NMR-Spektrum (DMSO + CD₃0D) Signale bei ppm: 3,15 (s,3H), 7,2 (s,1H), 7,8 (breites s,4H).

### Beispiel B

### 5-Amino-4-hydroxy-2-p-methylsulfinylanilino-pyrimidin

3,8 g (0,014 Mol) 4-Hydrpxy-2-p-methylthio-anilino-5-nitro- pyrimidin werden in 50 ml Eisessig aufgeschlämmt, 2,3 g (0,02 Mol) 30%iger Wasserstoffperoxid zugesetzt und unter Rühren 10 Minuten auf dem Dampfbad erwärmt. Nach dem Abkühlen wird der entstandene Niederschlag abgesaugt und im Vakuum getrocknet. Ausbeute: 2,28 g (55 % der Theorie) 4-Hydroxy-2-p-methyl- sulfinylanilino-5-nitro-pyrimidin. 2,1 g (0,0079 Mol) 4-Hydroxy-2-p-methylsulfinylanilino-5-nitropyrimidin werden in 60 ml Wasser aufgeschlämmt und durch Zusatz von 7 ml konzentriertem Ammoniak und Erwärmen gelöst. 8 g Natriumdithionit werden auf einmal zugegeben und die Lösung 20 Minuten auf dem Dampfbad erhitzt. Nach dem Abkühlen wird mit Eisessig die Lösung auf pH 7 gebracht und mehrere Stunden im Kühlschrank stehengelassen. Nach dem Absaugen wird das Rohprodukt durch Chromatographie über eine Kieselgel-Säule (Fließmittel Chloroform/Methanol 5:1) gereinigt. Ausbeute: 0,8 g (38,2 % der Theorie). NMR-Spektrum (DMSO + CD₃OD) Signale bei ppm: 2,75 (s,3H), 7,15 (s,1H), 7,7 (q,4H).

Analog, ausgehend von dem entsprechend substituierten Anilin und 4-Hydroxy-2-p-methylthio-anilino-5-nitro-pyrimidin bzw. 2-Chlor-4-hydroxy-5-nitropyrimidin und anschließender Reduktion wurden die in der folgenden Tabelle aufgeführten Pyrimidine synthetisiert.

Pyrimidine der allgemeinen Formel

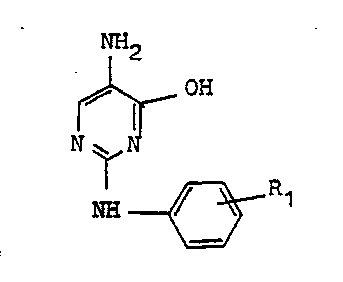

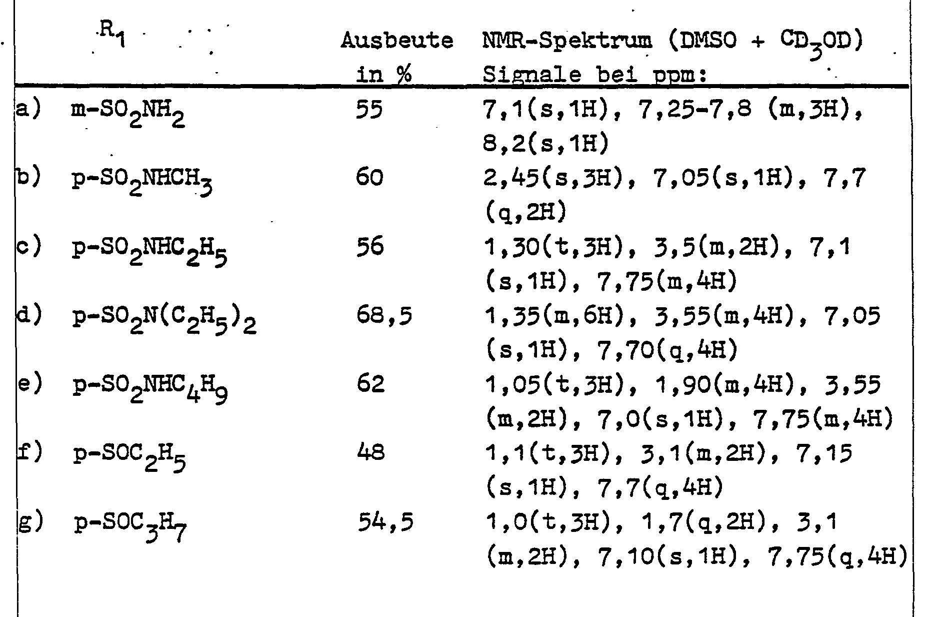

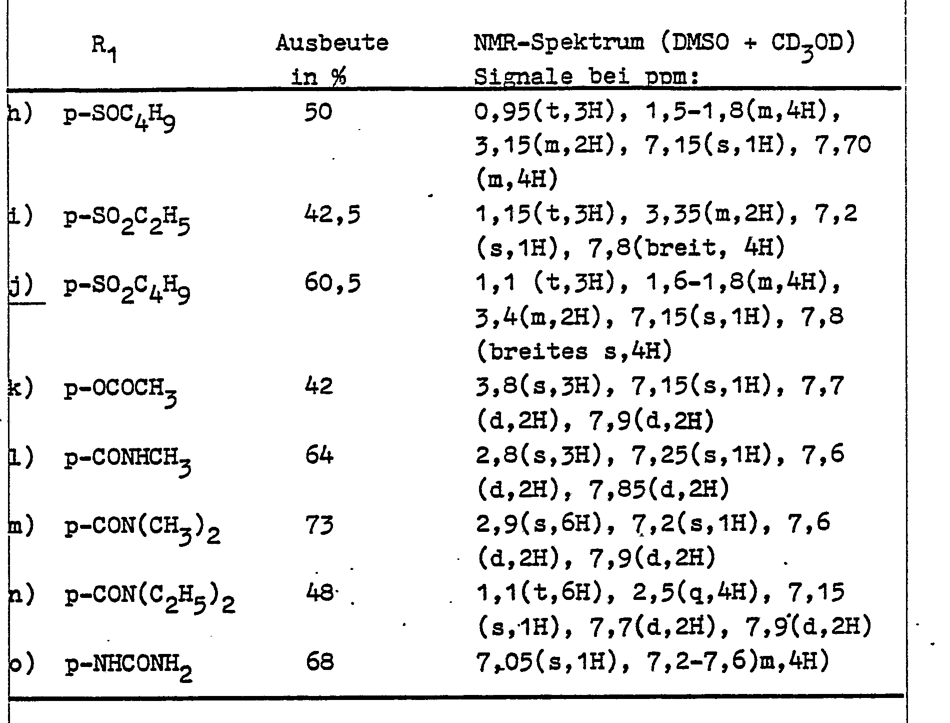

Pyrimidine der allgemeinen Formel

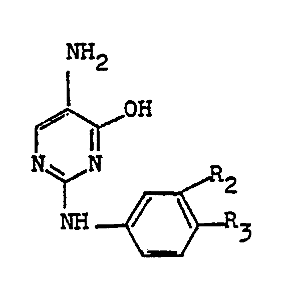

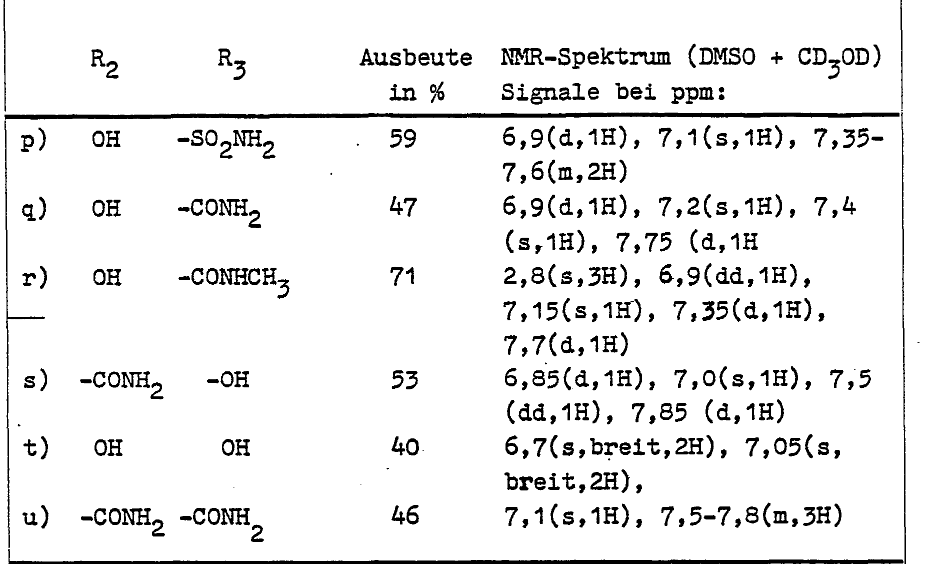

Pyrimidine der allgemeinen Formel

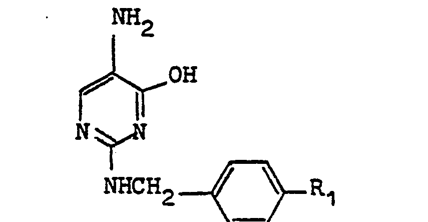

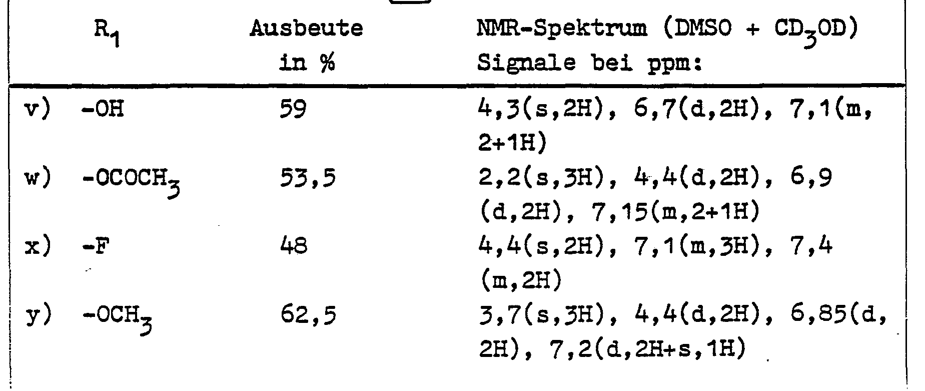

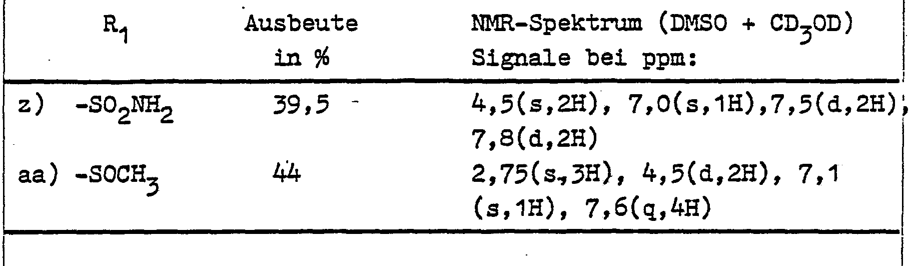

### Beispiel C

### D-L-α-[3-(4-Hydroxy-2-p-methylaminosulfonylanilino-5-pyrimidinyl)-ureido]-thienylessisgsäure

2,95 g (0,01 Mol) 5-Amino-2-p-methylaminosulfonylanilino-4-hydroxy-pyrimidin werden in 100 ml trockenem Tetrahydrofuran mit 6 g Trimethylsilyldiäthylamin 30 Minuten.lang zum Rückfluß erhitzt. Anschließend wird-im Vakkum zur Trockne eingeengt und das Festprodukt unter Stickstoff in 60 ml trockenem Tetrahydrofuran gelöst. Diese Lösung wird unter Eiskühlung zu 1,05 g Phosgen, gelöst in 50 ml Tetrahydrofuran getropft. Man rührt 5 Minuten bei Raumtemperatur nach und engt im Vakuum etwa auf das halbe Volumen ein. Diese Lösung wird unter Eiskühlung zu 1,57 g (0,01 Mol) D,L-Thienylglycin, das mit 1 n Natronlauge in einem Gemisch von 50 ml Tetrahydrofuran und 20 ml Wasser gelöst wurde, getropft. Der pH-Wert wird am Ende der Zugabe auf 8,0 eingestellt. Man rührt eine Stunde bei Raumtemperatur nach, zieht dann das Tetrahydrofuran im Vakuum ab, gibt etwas Wasser zu und schüttelt zweimal mit Essigester bei pH 7,0 aus. Die wäßrige Phase wird dann mit 2 n Salzsäure auf pH 2,7 gestellt. Das ausgefallene Produkt wird abgesaugt und getrocknet. Ausbeute: 3,3 g (69 %)

IR-Spektrum: 3300, 1660, 1555 cm⁻¹; NMR-Spektrum (DMSO + CD₃OD) Signale bei ppm: 2,45(s,3H), 5,50 (s,1H), 7,05(m,2H), 7,40 (m,1H), 7,80 (q,4H), 8,35(s,1H).

Analog wurden folgende Ureidocarbonsäuren synthetisiert:

### a) Ureidocarbonsäuren der allgemeinen Formel

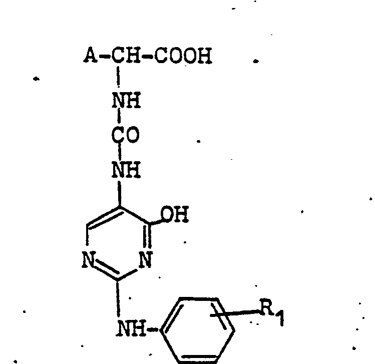

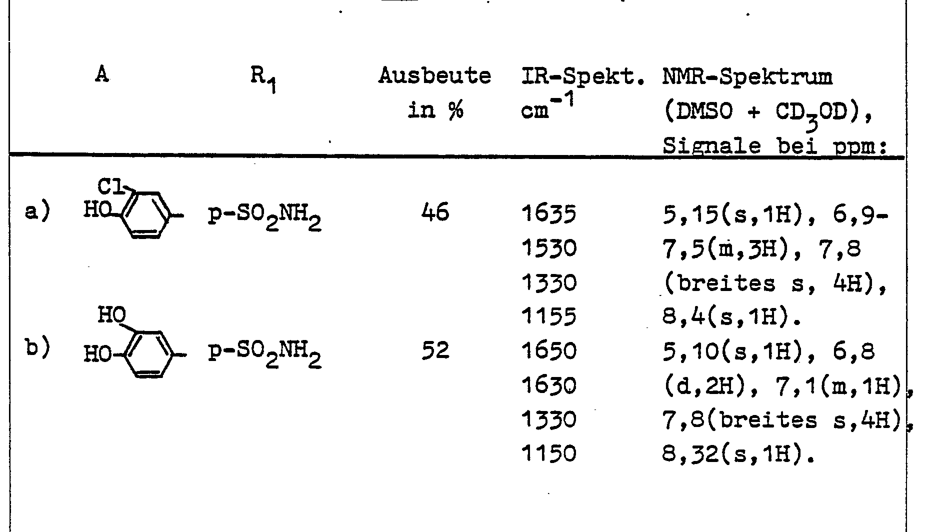

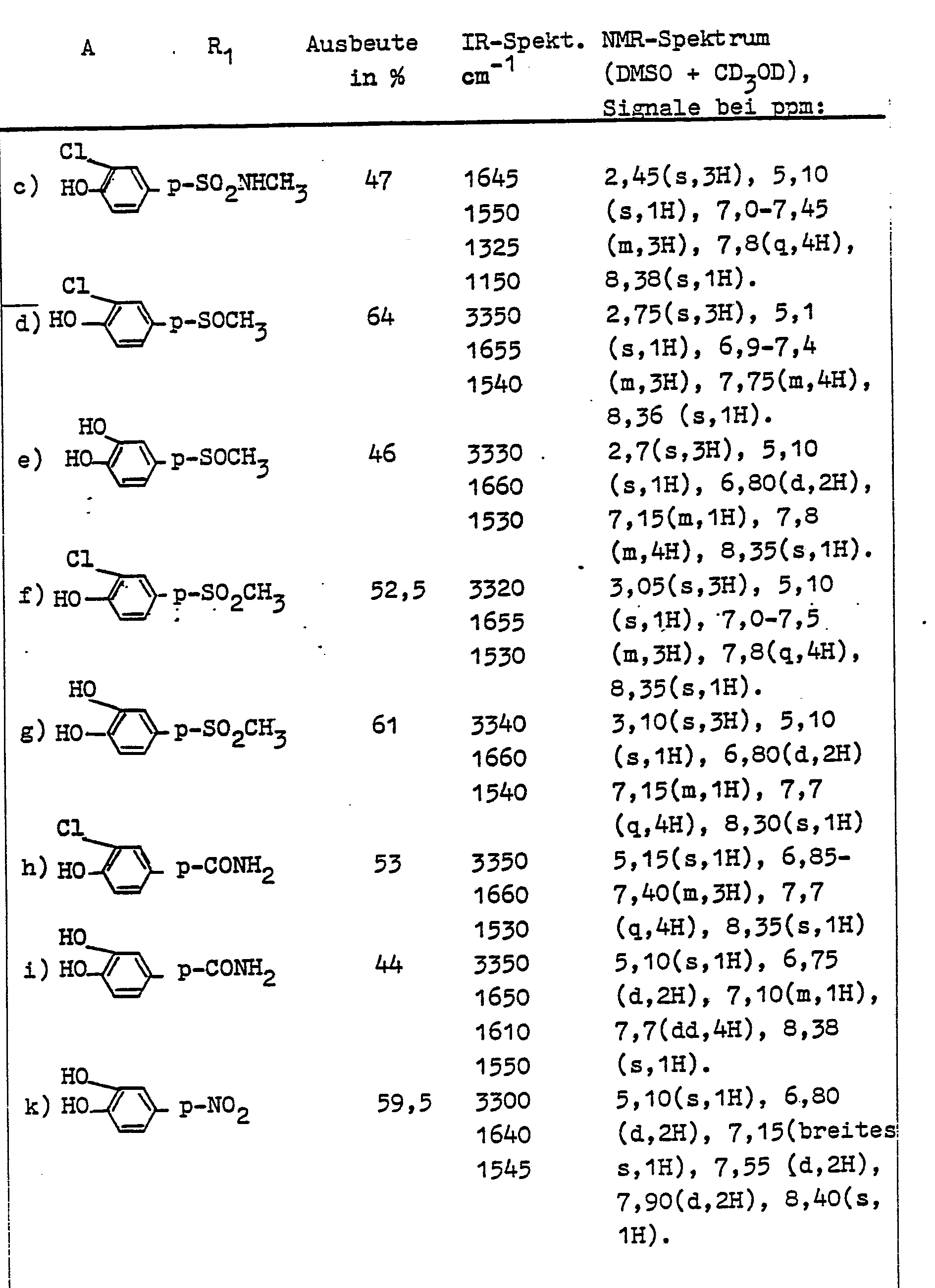

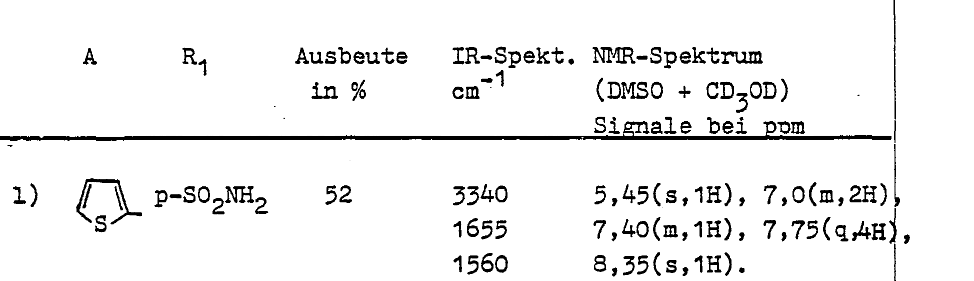

### b) Ureidocarbonsäuren der allgemeinen Formel

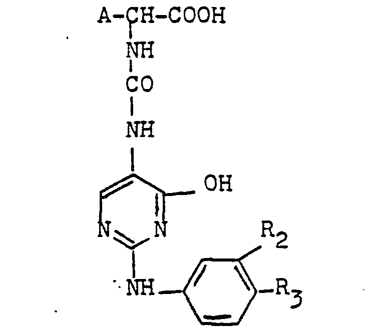

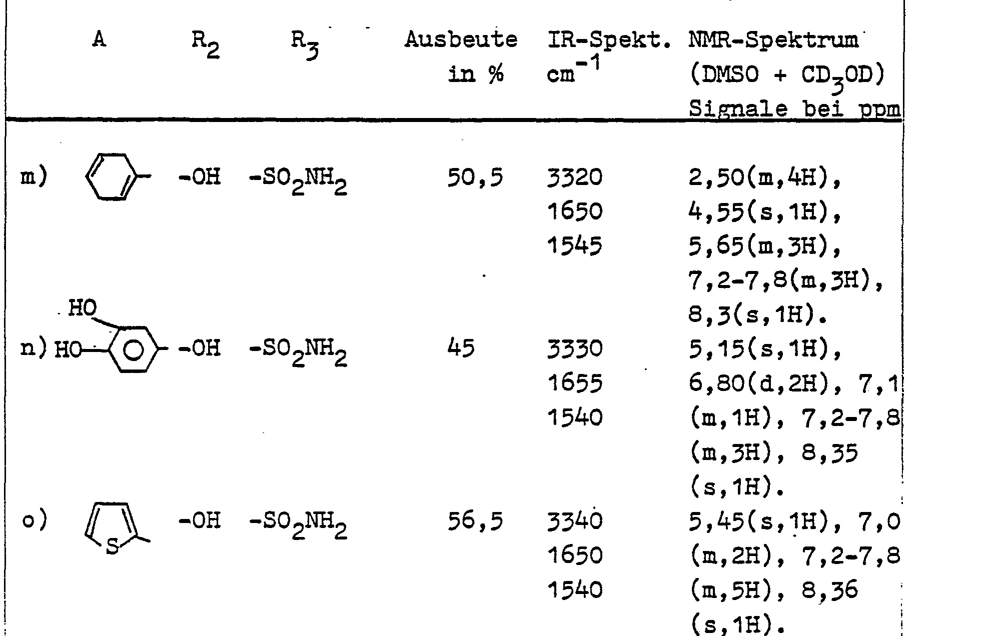

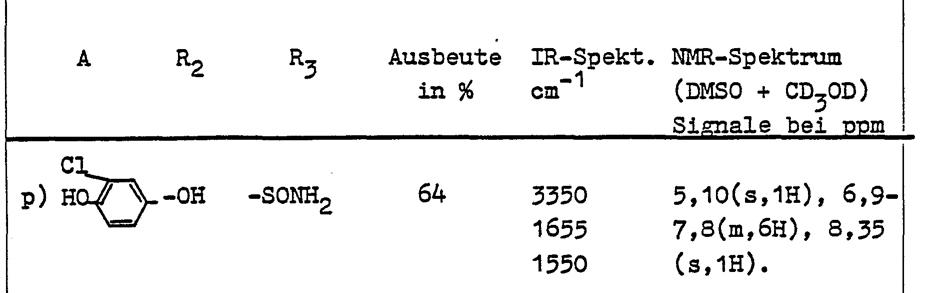

### c) Ureidocarbonsäuren der allgemeinen Formel

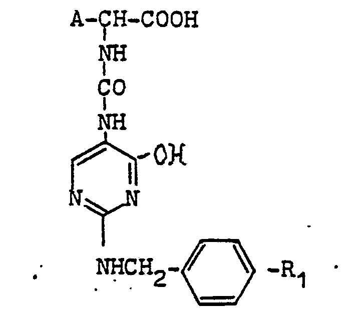

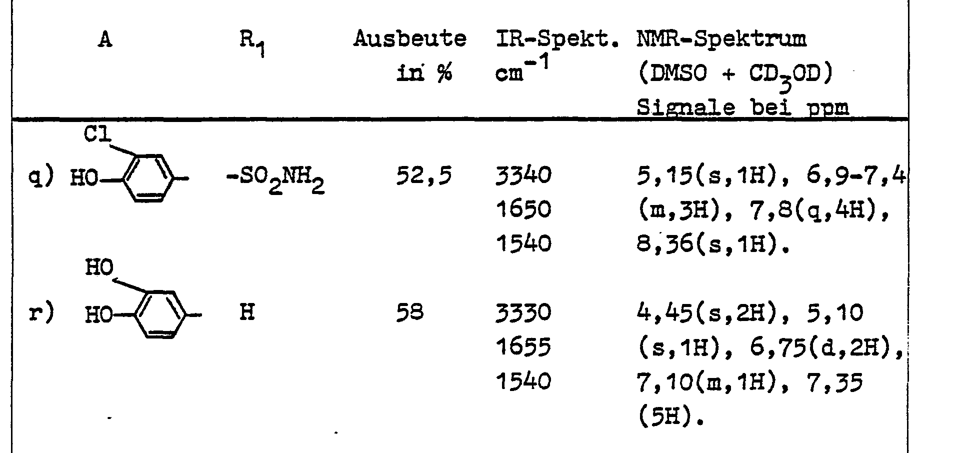

II Darstellung der Endprodukte

### Beispiel 1

D-α-[3-(4-Hydroxy-2-p-methylaminosulfonylanilino-5-pyrimidinyl)-ureido]-p-hydroxy-benzylpenicillin-Natrium

1,47 g (0,005 Mol) 5-Amino-4-hydroxy-2-p-methylaminosul- fonylanilino-pyrimidin werden in 50 ml trockenem Tetrahydrofuran aufgeschlämmt und mit 4 g Trimethylsilyldiäthylamin bis zur vollständigen Lösung zum Rückfluß erhitzt (10 bis 30 Minuten). Die Lösung wird im Vakuum zur Trockne eingeengt, wieder in 50 ml Tetrahydrofuran aufgenommen und unter Eiskühlung zu einer Lösung von 530 mg Phosgen in 35 ml trockenem Tetrahydrofuran zugetropft. Man rührt 10 Minuten bei Raumtemperatur nach und engt dann im Vakuum etwa zur Hälfte ein.

Man suspendiert 2,1 g (0,005 Mol) Amoxicillin in einem Lösemittelgemisch von 40 ml Tetrahydrofuran und 10 ml Wasser. Unter Eiskühlung fügt man bis zur Lösung Triäthylamin zu. Zu dieser Lösung wird die oben hergestellte Lösung zugetropft, wobei der pH-Wert durch Zugabe von Triäthylamin auf etwa 7,0 gehalten wird. Man rührt eine Stunde im Eisbad nach, läßt auf Raumtemperatur kommen, versetzt mit 20 ml Wasser und entfernt das Tetrahydrofuran im Vakuum. Die wäßrige Phase wird einmal mit Essigester bei pH 7,0 ausgeschüttelt und unter Eiskühlung mit 1n Salzsäure auf pH 2,8 gestellt. Das ausgefallene Penicillin wird abgesaugt und im Vakuum getrocknet.

Durch Zusatz der berechneten Menge Natrium-äthylhexanoat in Methanol wird das Natriumsalz hergestellt und mit Äther ausgefällt.

Ausbeute: 2,16 g (62 %);

IR-Spektrum: 1765, 1650, 161₀ cm⁻¹; NMR-Spektrum (DMSO + CD₃OD) Signale bei ppm: 1,55 (d,6H), 2,55(s,3H), 4,05 (s,1H), 5,45 (d+s,3H), 6,75 (d,2H), 7,30 (d,2H), 7,85 (q,4H), 8,35 (s,1H).

Nach dieser Methode wurden die Penicilline der folgenden Formel synthetisiert:

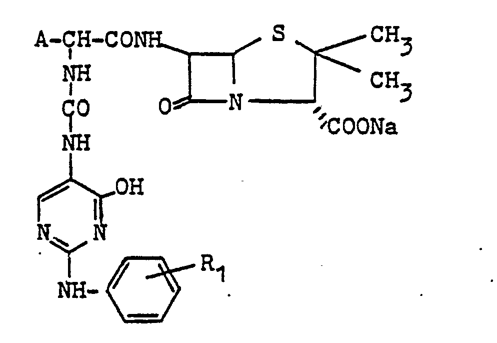

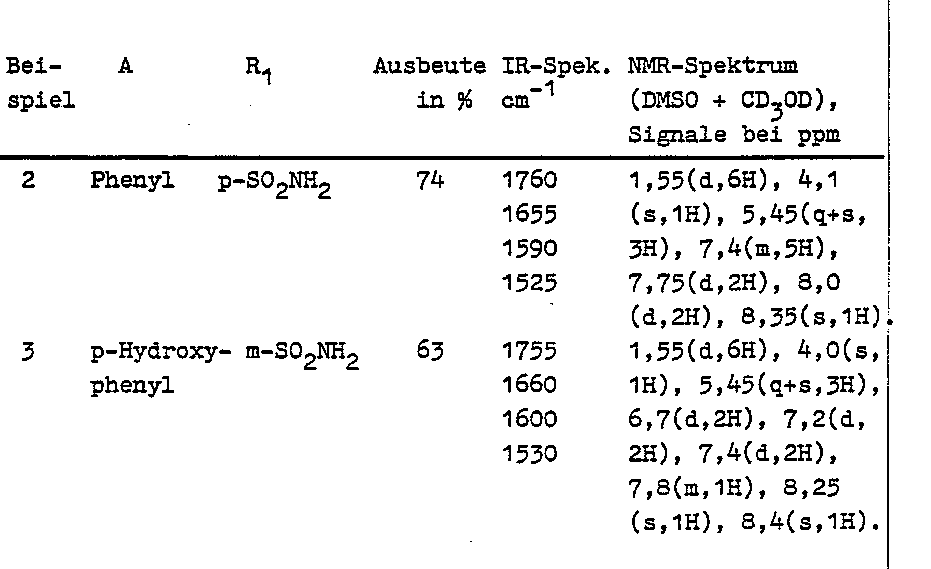

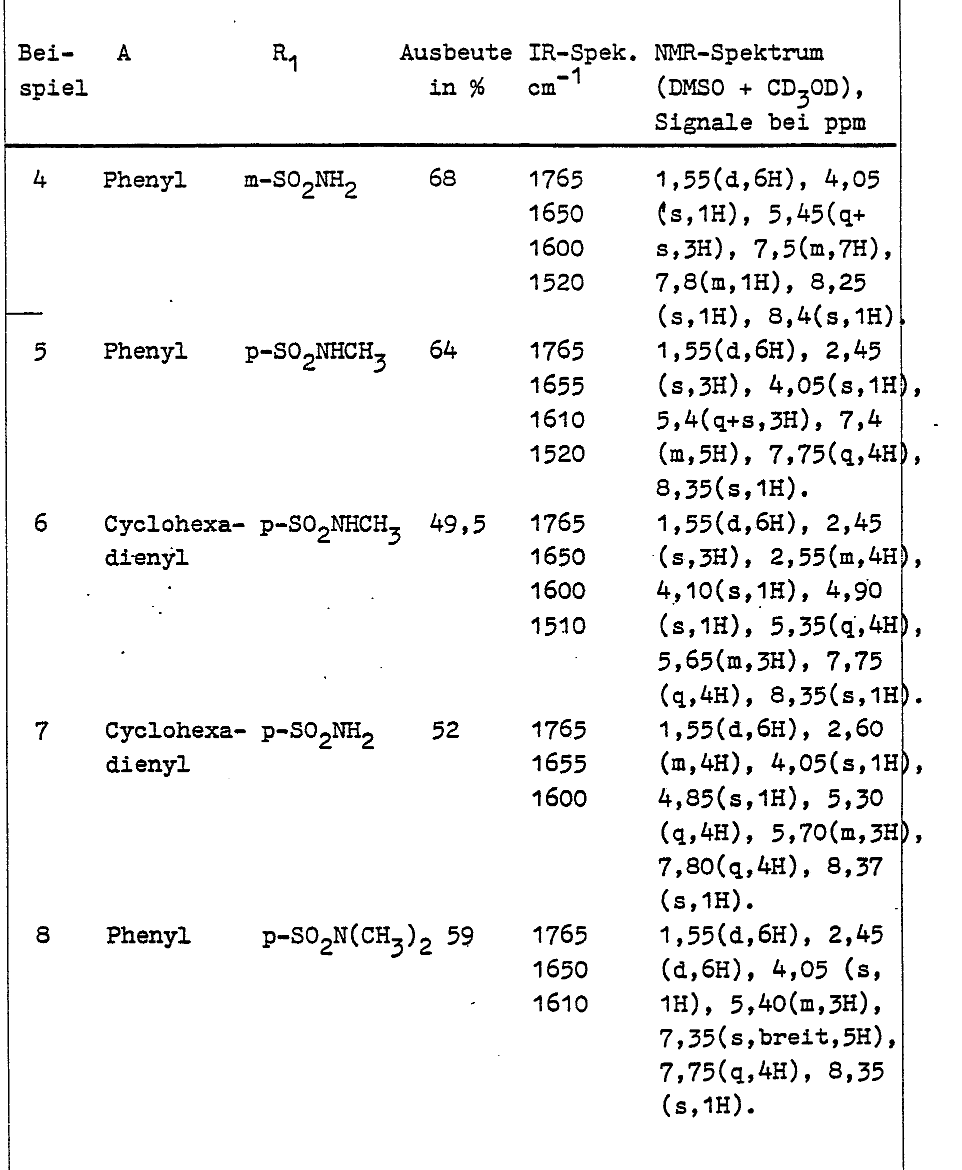

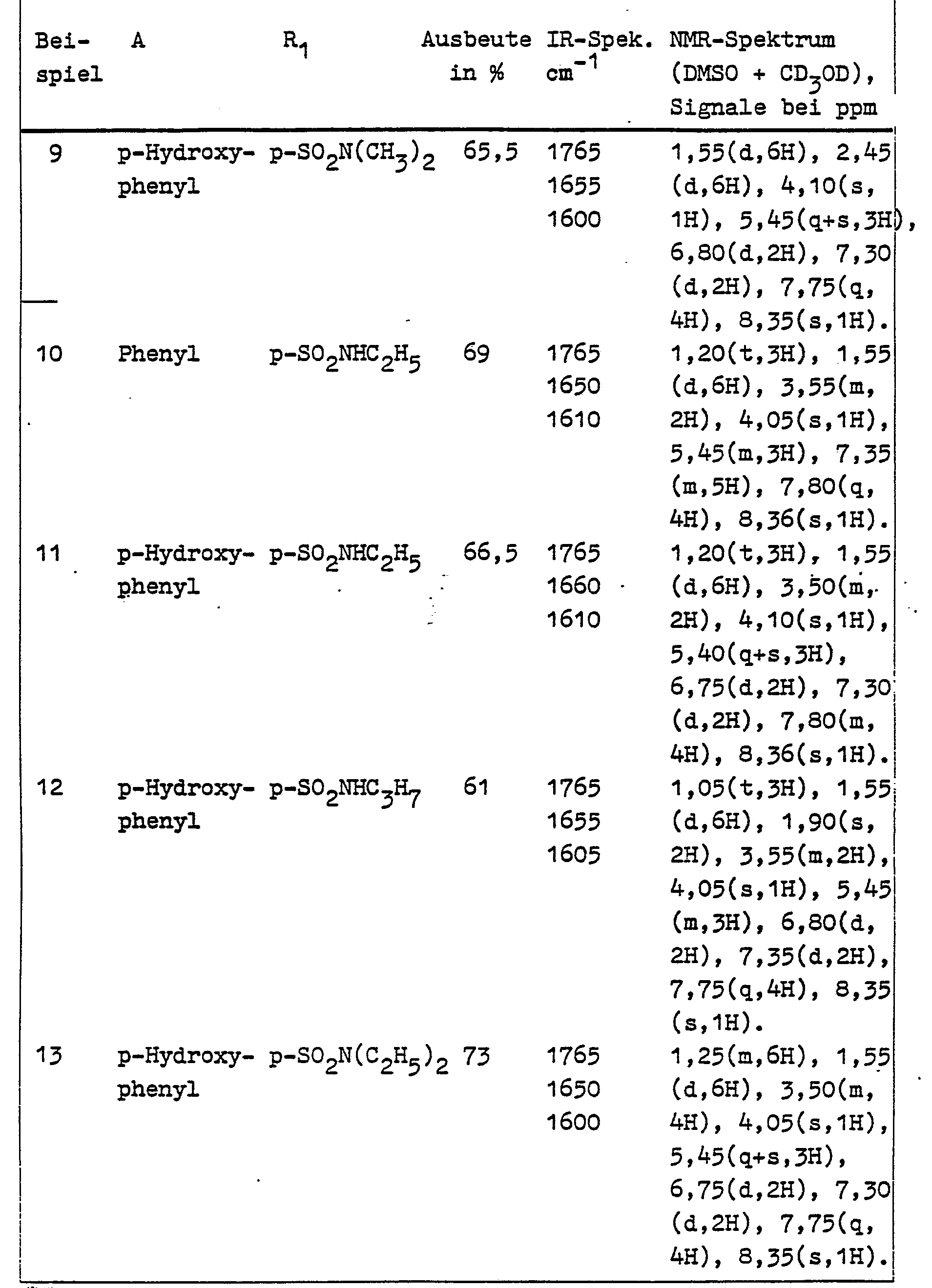

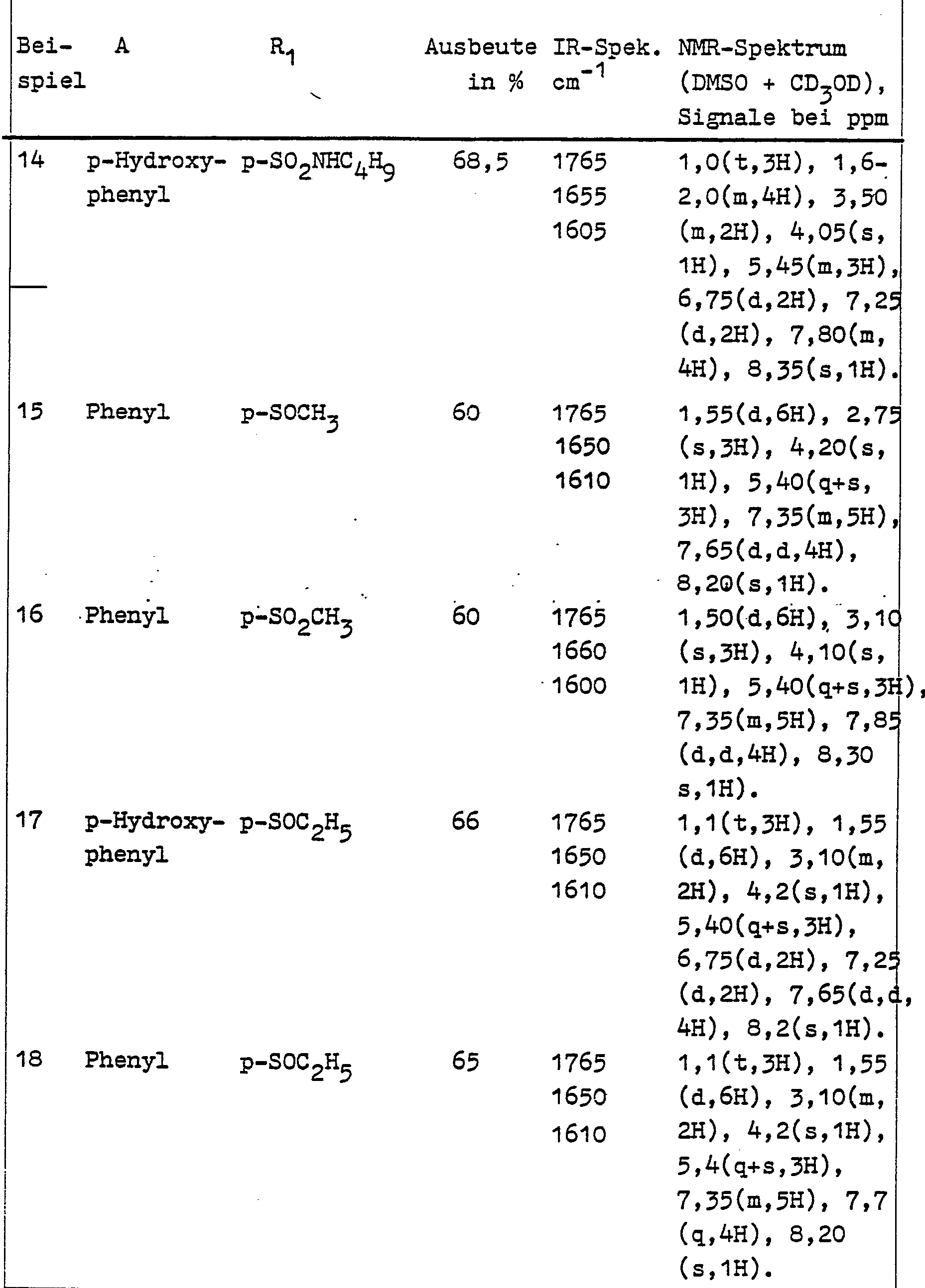

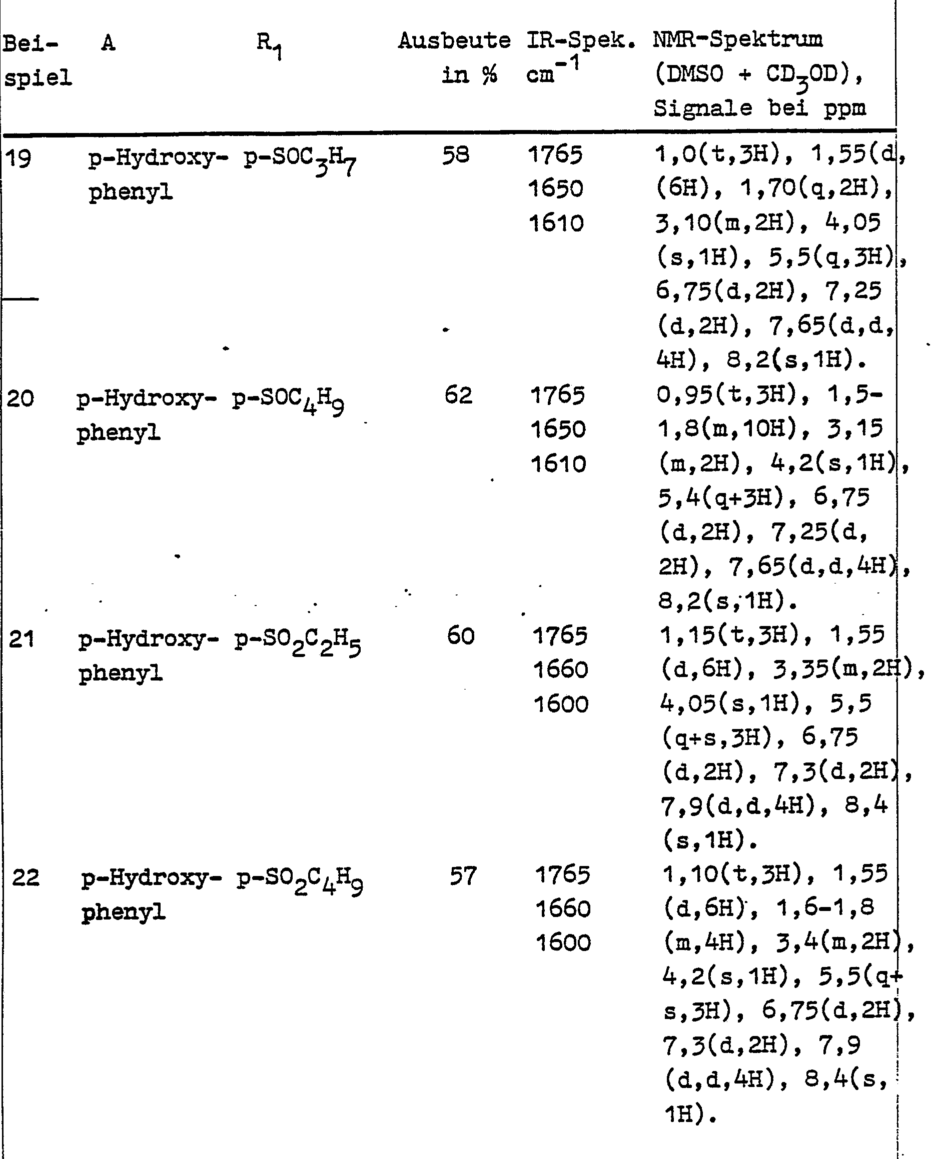

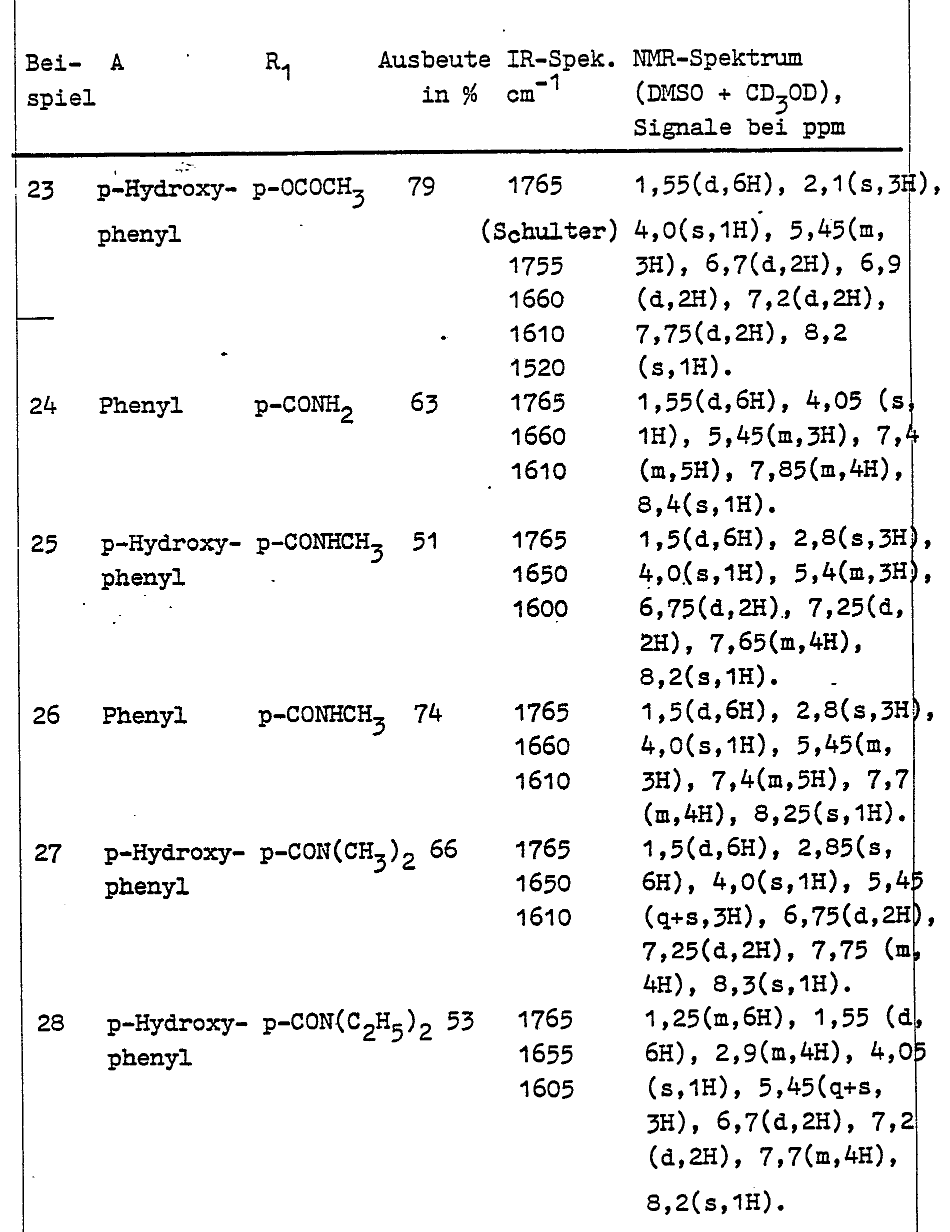

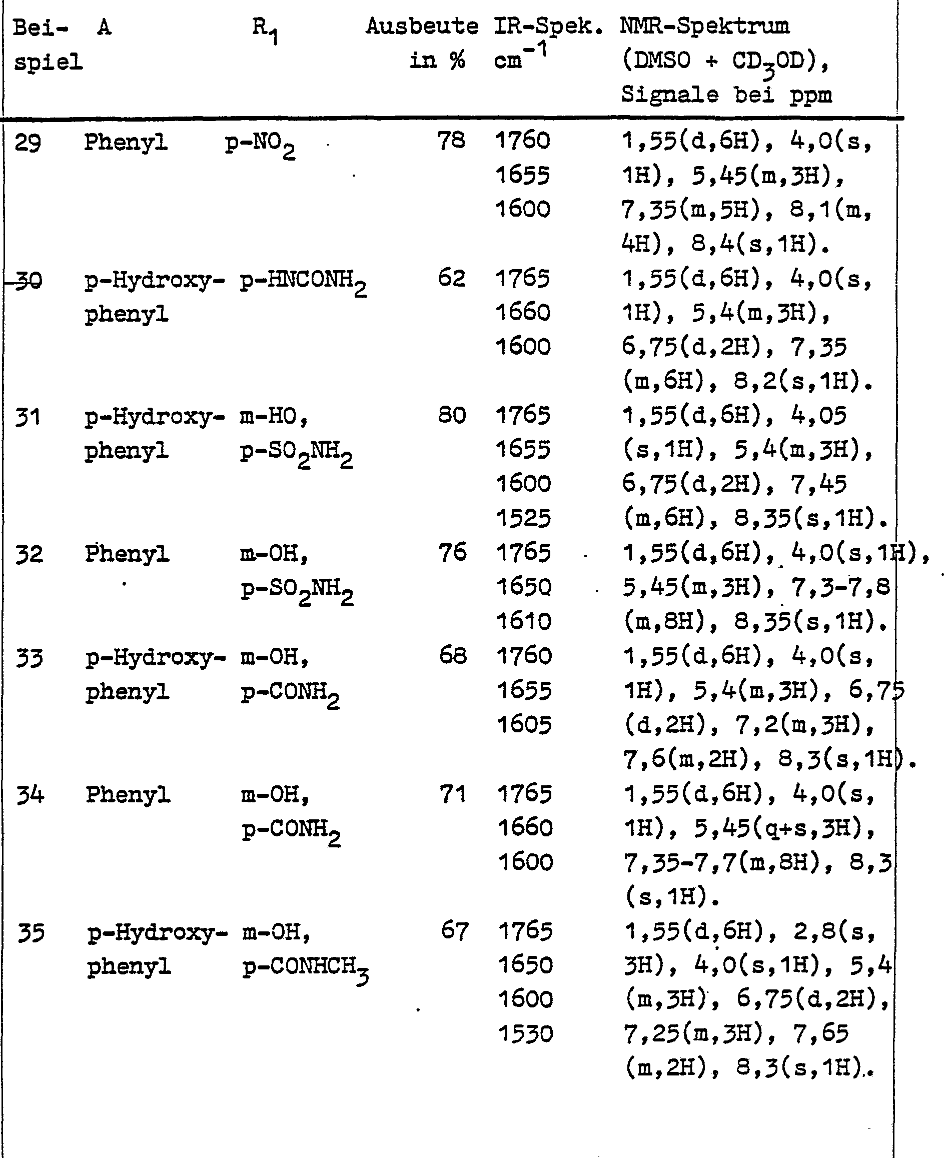

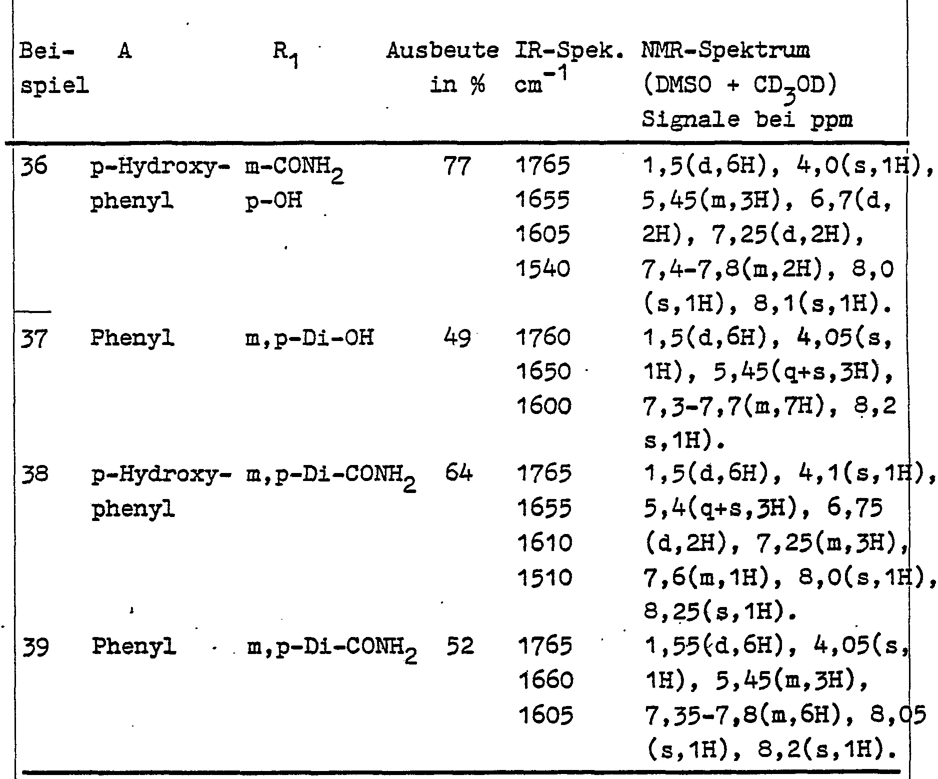

Des weiteren wurden Penicilline der Formel

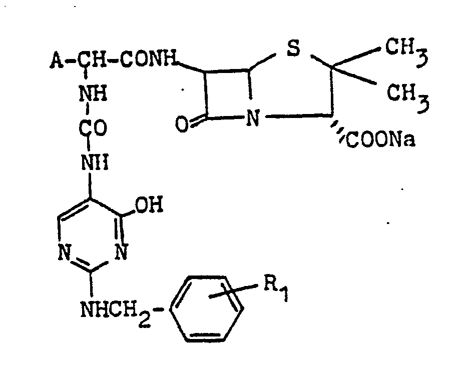

in analoger Weise hergestellt:

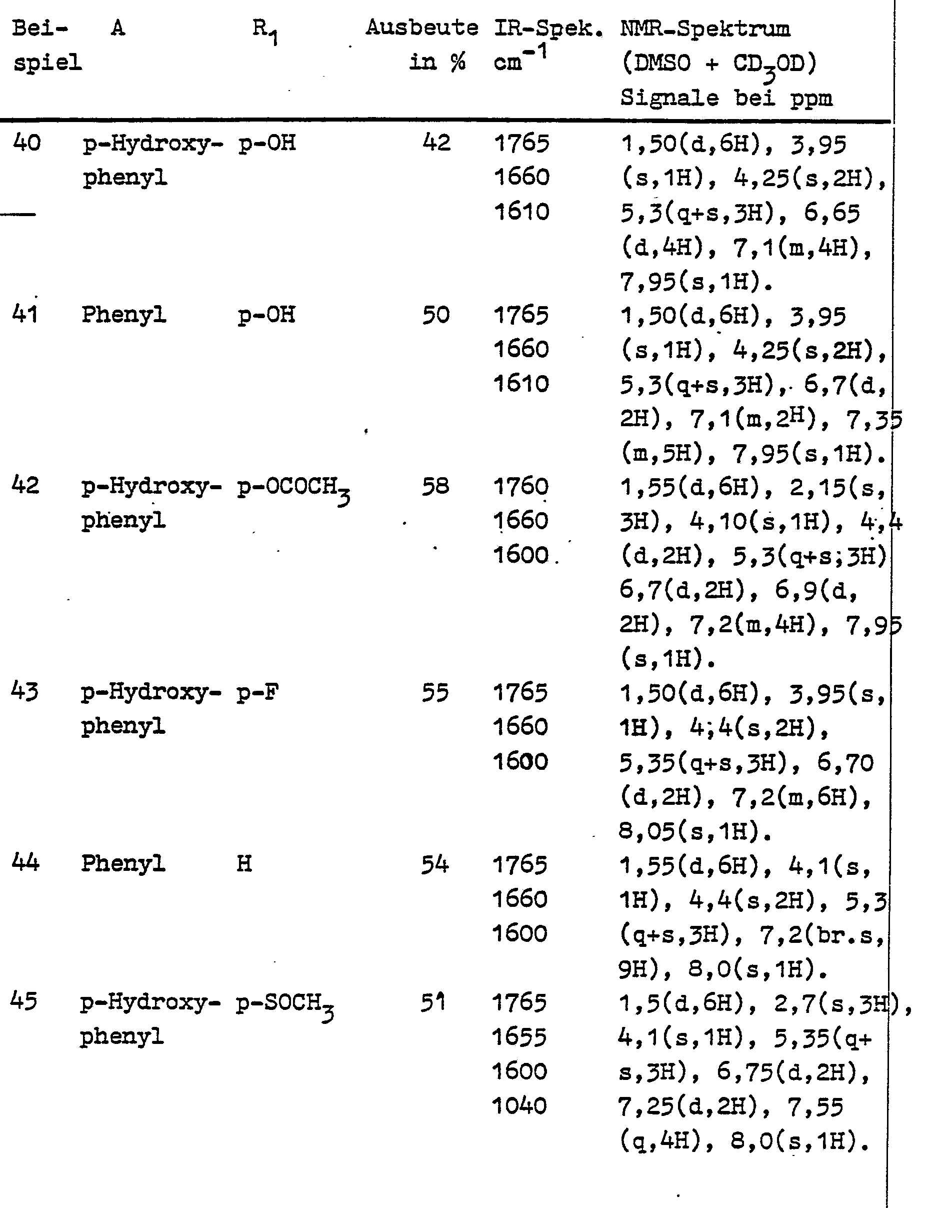

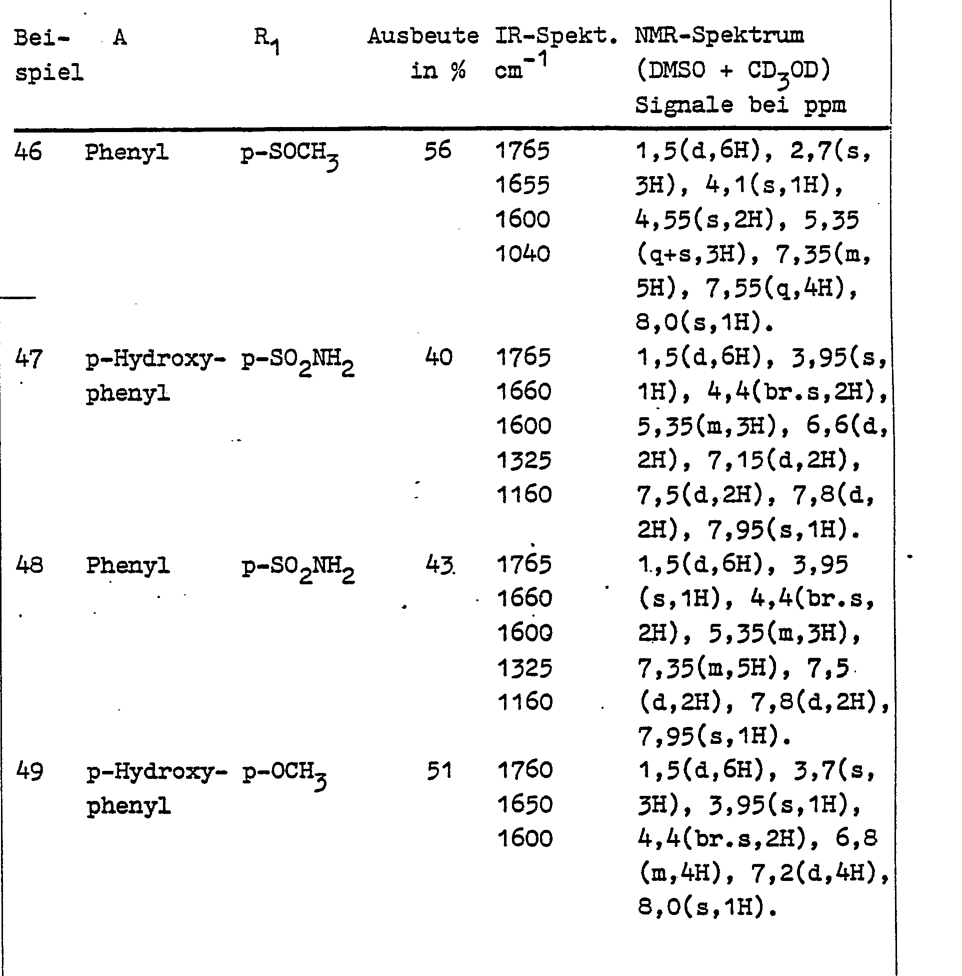

### Beispiel 50

D,L-α-[3-(2-p-Aminosulfonylanilino-4-hydroxy-5-pyrimidinyl)-ureido]-2-thienylmethyl-penicillin-Natrium

Eine Lösung von 2,3 g ( 5 mmol) D,L-α-[3-(2-p-Aminosulfonyl- anilino-4-hydroxy-5-pyrimidinyl)-ureido]-thienylessigsäure in 50 ml Dimethylformamid und 25 ml Methylenchlorid wird mit 0,52 g (5,1 mmol) N-Methylmorpholin versetzt und auf -20° bis 25°C abgekühlt.

Hierzu gibt man 0,54 g (5mmol) Chlorameisensäureäthylester, gelöst in 20 ml Methylenchlorid und rührt die Mischung 45 Minuten bei -20°. (Lösung A).

Zu einer Suspension von 1,1 g ( 5 mmol) 6-Aminopenicillansäure in 50 ml Methylenchlorid werden 3,1 g (30 mmol) Triäthylamin getropft, danach 1-2.Stunden bei Raumtemperatur bis zur Lösung gerührt und dam auf -20°C abgekühlt (Lösung B).

Man gibt Lösung B zur Lösung A, wobei die Temperatur auf -20°C gehalten wird, und läßt die Reaktionsmischung 45 Minuten bei -20° und 2 Stunden bei Raumtemperatur nachreagieren.

Anschließend werden 150 ml Wasser zugegeben, der pH-Wert der wäßrigen Phase auf 7,2 gestellt, die organische Schicht abgetrennt und die Wasserphase 3 mal mit Essigester extrahiert. Die Essigester-Extrakte werden verworfen. Man überschichtet die wäßrige Phase mit Essigester und stellt deren pH durch Zugabe von 2 n Salzsäure auf 3,0 ein.

Die organische Schicht wird abgetrennt, mit Natriumsulfat getrocknet und eingeengt. Man löst das erhaltene Penicillin in Methanol und fällt durch Zugabe einer äquimolaren Menge Natrium-äthylhexanoat das Natriumsalz aus.

Ausbeute: 2,9 g (84 % der Theorie), IR-Spektrum: 1760, 1655, 1600 cm⁻¹, NMR-Spektrum (DMSO + CD₃0D) Signale bei ppm: 1,55(d,6H), 4,0(s,1H), 5,45(m,3H), 7,1(m,2H), 7,45(m,1H), 7,85(q,4H), 8,35(s,1H).

Nach der Darstellungsmethode des Beispiels 50 wurden folgende Penicilline synthetisiert:

### ä) der Formel

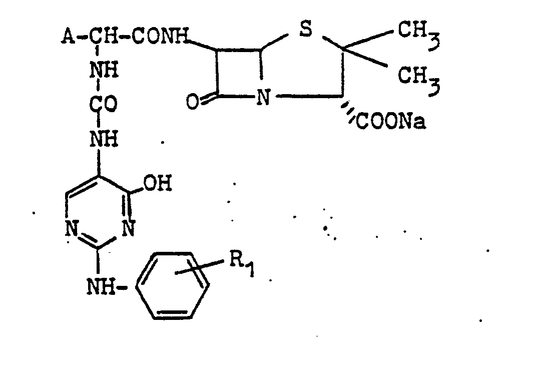

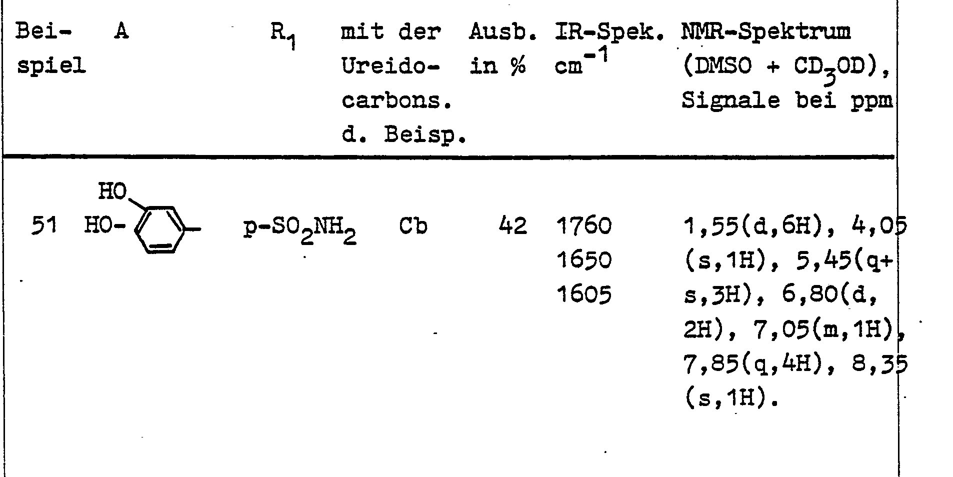

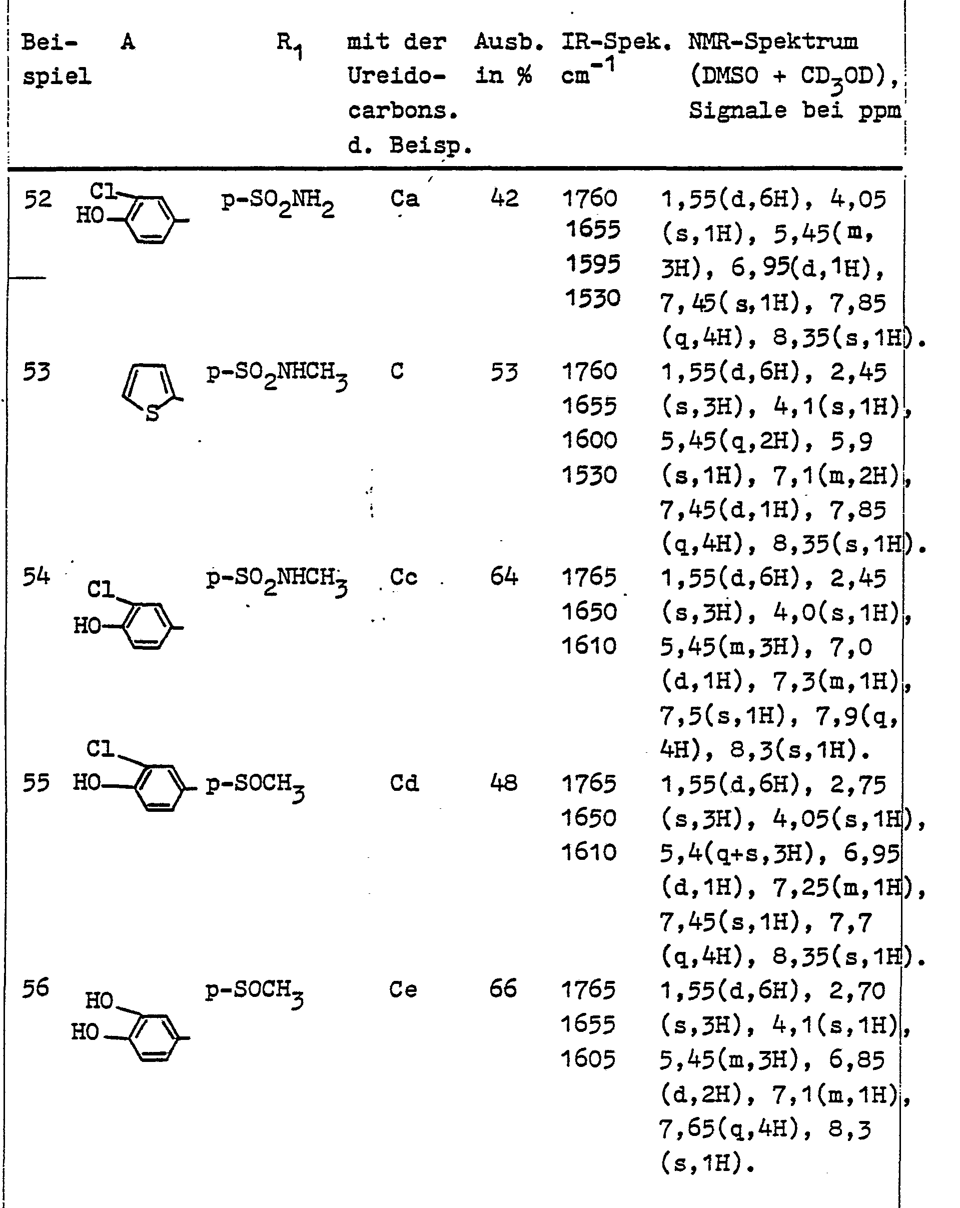

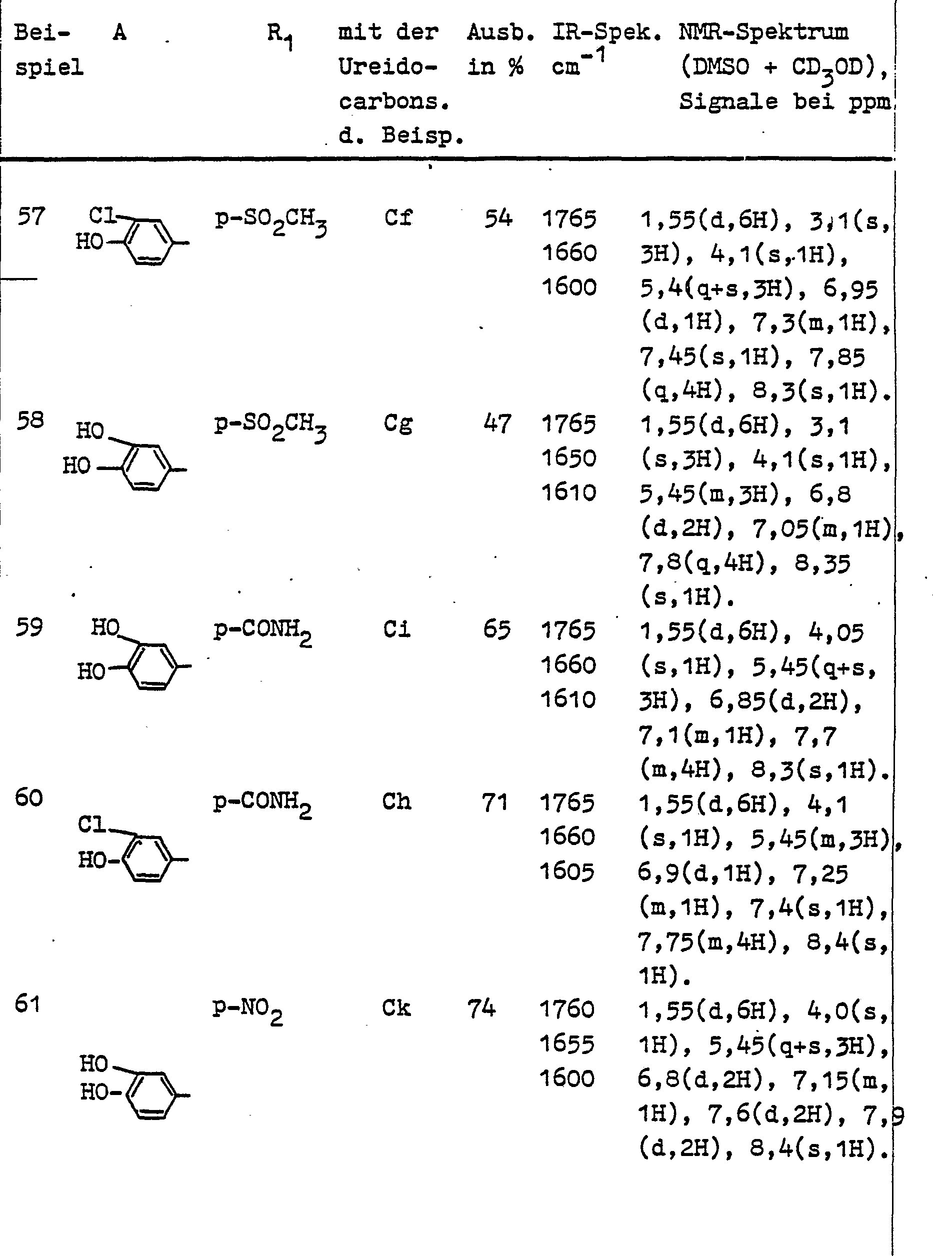

### b) der Formel

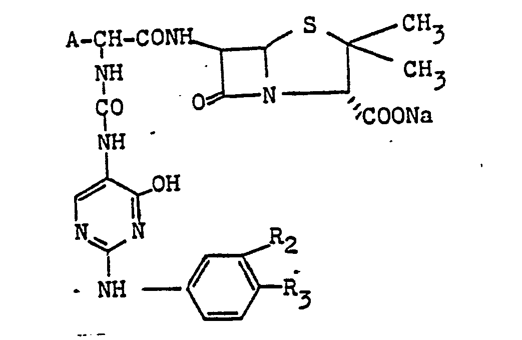

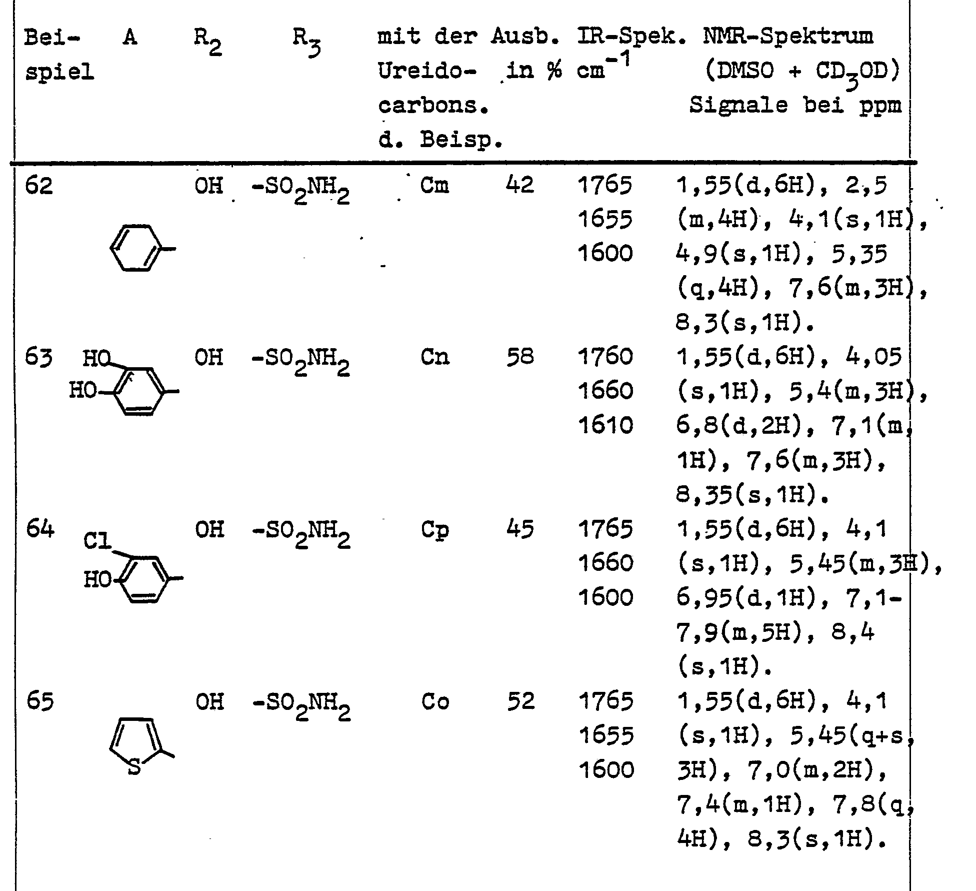

### c) der Formel

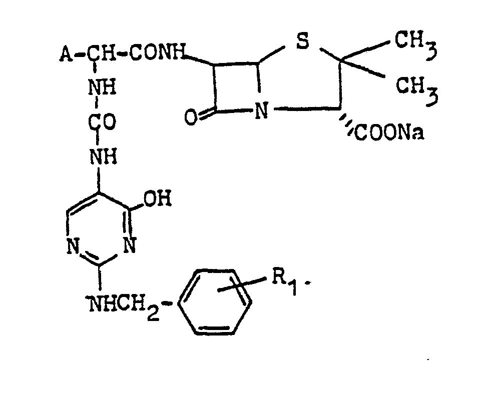

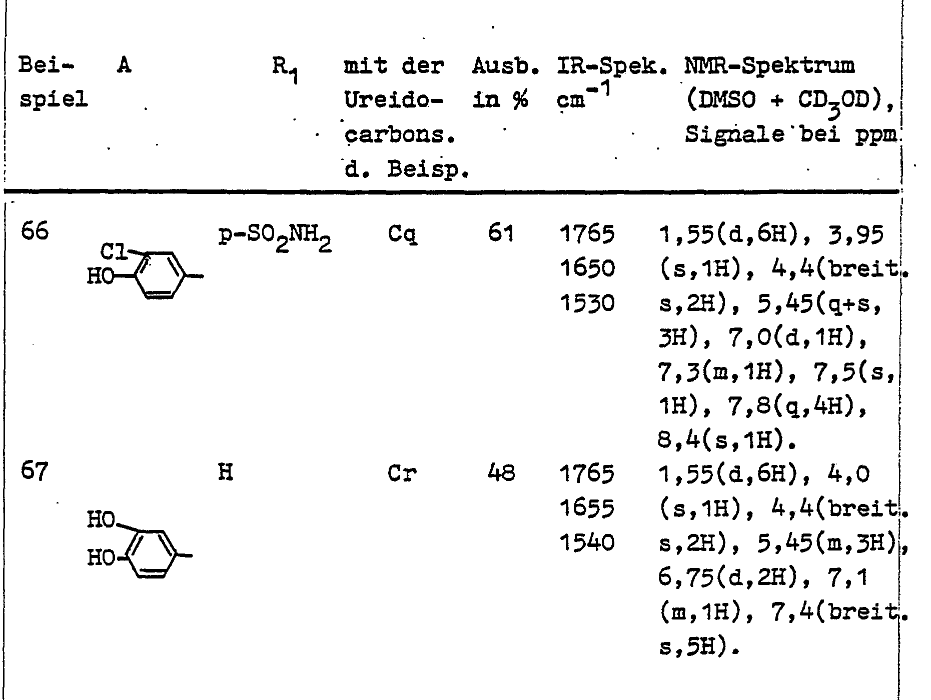

Die Verbindungen der allgemeinen Formel I und I' lassen sich in die üblichen pharmazeutischen Anwendungsformen, wie Tabletten, Dragees, Kapseln oder Ampullen einarbeiten. Die Einzeldosis beträgt bei Erwachsenen im allgemeinen zwischen 50 und 1000 mg, vorzugsweise 100 bis 500 mg, die Tagesdosis zwischen 100 und 4000 mg, vorzugsweise 250 bis 2000 mg.

### Beisniel I

Tabletten enthaltend D-α-[3-(2-p-Aminosulfonylanilino-4-hydroxy-5-pyrimidinyl)-ureido]-benzylpenicillin-Natrium

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

### Beispiel II

Dragees enthaltend D-α-[3-(2-p-Aminosulfonylanilino-4-hydroxy-5-pyrimidinyl)-ureido]-benzylpenicillin-Natrium

Analog Beispiel I werden Tabletten gepreßt, die anschließend in üblicher Weise mit einem Überzug bestehend aus Zucker, Kartoffelstärke, Talk und Tragant überzogen werden.

### Beispiel III

Kapseln enthaltend D-α-[3-(2-p-Aminosulfonylanilino-4-hydroxy-5-pyrimidinyl)-ureido]-benzylpenicillin-Natrium

5 kg Wirksubstanz werden in üblicher Weise in Hartgelatinekapseln gefüllt, derart, daß jede Kapsel 500 mg des Wirkstoffes enthält.

### Beispiel IV

Trockenampullen enthaltend D-α-[3-(2-p-Aminosulfonylanilino-4-hydroxy-5-pyrimidinyl)-ureido]-benzylpenicillin-Natrium

In einem aseptischen Bereich wurde 251 g Wirkstoff in 200 ml destilliertem Wasser zur Injektion aufgelöst. Die Lösung wurde durch ein Millipore-Filter (Porengröße 0,22 mm, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragees, Kapseln und Ampullen erhältlich, die einen oder mehrere der übrigen Wirkstoffe der Formel I oder die physiologisch unbedenklichen Salze dieser Verbindungen enthalten.

## Patentansprüche

1) Neue Penicilline der allgemeinen Formeln 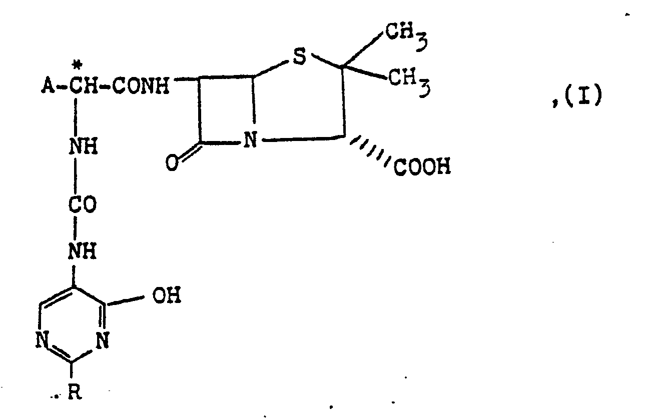 _{b}zw. 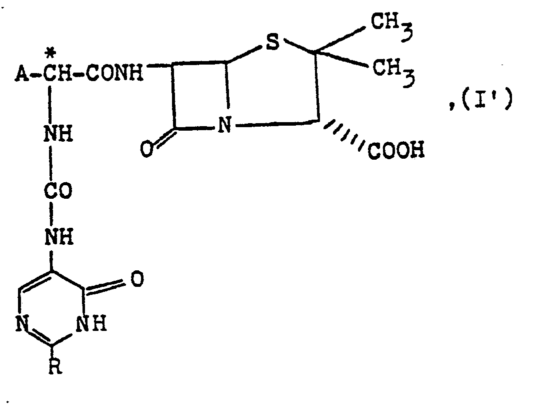 in welchen A und R die folgenden Bedeutungen besitzen:
a) A die Phenylgruppe und R die Gruppe der allgemeinen Formel II, 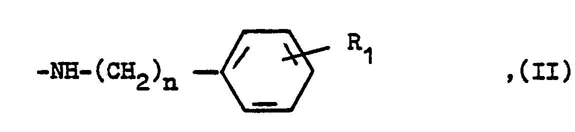 in der n=0 oder 1 ist und R₁, sofern n-0 ist, die Gruppen 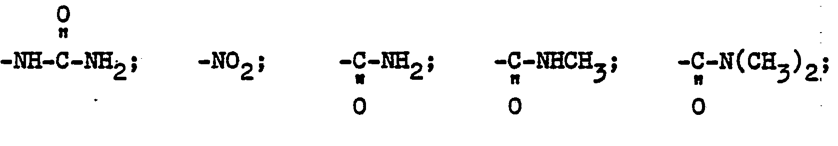 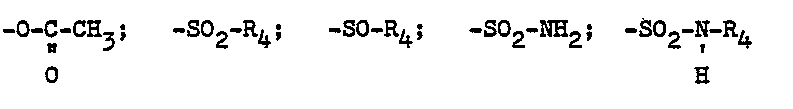 oder -SO₂-N(R₄)₂ bedeutet und, sofern n=1 ist, bedeutet es die oben genannten Gruppen und noch zusätzlich ein Wasserstoff- oder Fluoratom, die Hydroxy-, Methyl- oder. Methoxygruppe, wobei R₄ in den obigen Formeln eine Alkylgruppe mit 1 bis 4 Kohlenstoffatomen bedeutet, oder R eine Gruppe der allgemeinen Formel III, 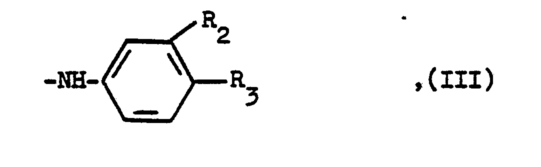 in der einer der Reste R₂ oder R₃ die Hydroxygruppe bedeutet und der andere dieser Reste eine Gruppe der Formeln 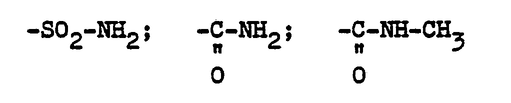 oder die beiden Reste R₂ und R₃ die Gruppe -CONH₂ darstellen, oder es bedeutet
b) A die Hydroxyphenylgruppe und
R die Gruppe der allgemeinen Formel II, in der n=0 oder 1 ist und R₁, sofern n=0 ist, die Gruppen der Formeln 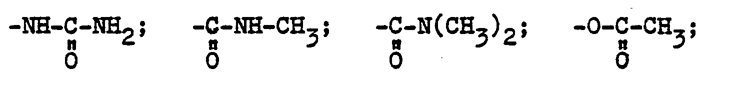 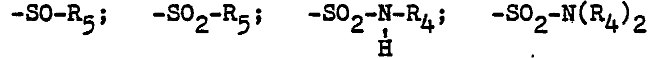 oder die meta-ständige Gruppe -S0₂-NH₂ bedeutet, und, sofern n=1 ist, bedeutet es die obengenannten Gruppen und noch zusätzlich ein Fluoratom, die Hydroxygruppe, die Nitrogruppe, die Gruppe H₂NCO-, die Gruppen -SO-CH₃, -SO₂-CH₃, oder p-H₂N-SO₂-, oder die Methyl- oder Methoxygruppe _{;} wobei in den obigen Formeln R₄ wie weiter oben angegeben definiert ist und R₅ eine Alkylgruppe mit 2 bis 4 Kohlenstoffatomen bedeutet, oder
R eine Gruppe der allgemeinen Formel III, in der einer der Reste R₂ oder R₃ die Hydroxygruppe bedeutet und der andere dieser Reste eine Gruppe der Formeln 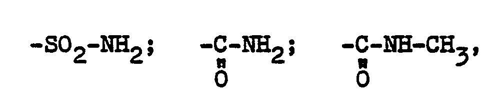 oder in der die beiden Reste R₂ und R₃ die Gruppe -CONH₂ darstellen, oder es bedeutet
c) A die 2-Thienylgruppe, die Cyclohexa-1,4-dien-1-ylgruppe, die 3,4-Dihydroxyphenyl- oder die 3-Chlor-4-hydroxyphenylgruppe und
R eine Gruppe der allgemeinen Formel II, in der n=0 oder 1 ist und R₁ ein Fluoratom, die Methyl- oder Methoxygruppe oder eine Gruppe der Formeln 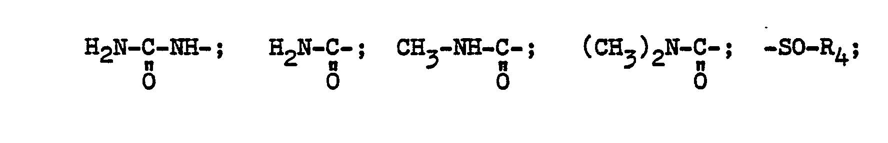 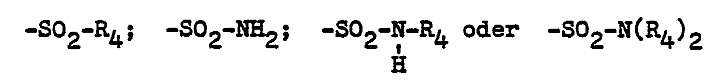 darstellt, wobei in diesen Formeln R₄ wie oben angegeben definiert ist, und R₁ auch ein Wasserstoffatom sein kann, sofern dann A die 2-Thienyl-, 3,4-Dihydroxyphenyl- oder 3-Chlor-4-hydroxyphenylgruppe bedeutet oder R₁ die Hydroxygruppe oder die Methylcarbonyloxygruppe, sofern A die 2-Thienylgruppe, die Cyclohexa-1,4-dien-1-ylgruppe oder die 3-Chlor-4-hydroxyphenylgruppe darstellt, oder
R eine Gruppe der allgemeinen Formel III, in der R₂ die Hydroxygruppe und R₃ die Hydroxygruppe oder die Gruppen -CONH₂, ₋CO-NHCH₃, -SO₂-NH₂ bedeuten, beide Reste R₂ und R₃ können auch gleichzeitig die Gruppe -CONH₂ bedeuten und ihre physiologisch verträgliche Salze mit anorganischen oder organischen Basen.

2) Neue Penicilline der allgemeinen Formel I bzw. I' gemäß Anspruch 1, dadurch gekennzeichnet, daß R_{.}die Gruppen der Formeln 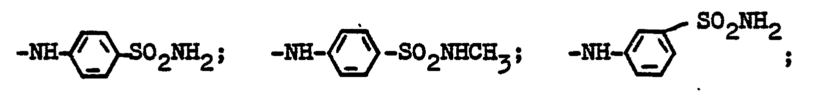 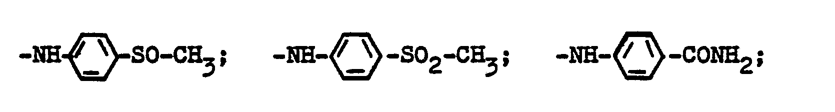 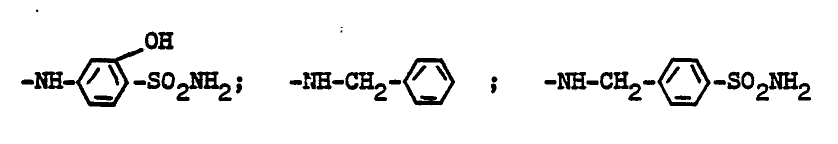 oder 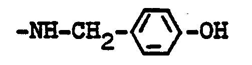 bedeutet und A wie im Anspruch 1 definiert ist, und ihre physiologisch verträgliche Salze mit anorganischen oder organischen Basen.

3) Neue Penicilline der allgemeinen Formel I bzw. I' gemäß Anspruch 1, dadurch gekennzeichnet, daß
a) R die Gruppe 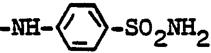 und A die Phenyl-, 2-Thienyl-, 3-Chlor-4-hydroxyphenyl- oder die 3,4-Dihydroxyphenyl- : gruppe oder die 1,4-Cyclohexadien-1-ylgruppe oder
b) R die Gruppe 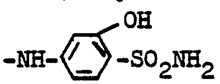 und A die Phenyl-, p-Hydroxyphenyl-, 2-Thienyl-, 3-Chlor-4-hydroxyphenyl-oder 3,4-Dihydroxyphenylgruppe, oder
c) R die Gruppe 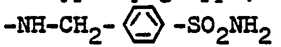 und A die Phenyl- oder p-Hydroxyphenylgruppe bedeutet, und ihre physiologisch ― verträgliche Salze mit anorganischen oder organischen Basen.

4) Als neue Verbindungen ;
D-α-[3-( 2-p-Aminosulfonylanilino-4-hydroxy-5-pyrimidinyl)-ureidp]-benzylpenicillin-Natrium
D,L-α-[3-(2-p-Aminosulfonylanilino-4-hydroxy-5-pyrimidinyl)-ureido]-2-thienylmethyl-penicillin-Natrium
D,L-α-[3-(2-p-Aminosulfonylanilino-4-hydroxy-5-pyrimidinyl)-ureido7-m,p-dihydroxy-benzylpenicillin-Natrium
D,L-α-[3-(2-p-Aminosulfonylanilino-4-hydroxy-5-pyrimidinyl)-ureido7-m-chlor-p-hydroxy-benzylpenicillin-Natrium
D-α-[3-(2-p-Aminosulfonylamilino-4-hydroxy-5-pyrimidinyl)-ureido]-1,4-cyclohexadien-1-yl-methylpenicillin-Natrium
D-α-[3-(4-Hydroxy-2-p-methylaminosulfonylanilino-5-pyrimidinyl)-ureido]-p-hydroxy-benzylpenicillin-Natrium
D-α-[3-(4-Hydroxy-2-p-methylaminosulfonylanilino-5-pyrimidinyl)-ureido7-benzylpenicillin-Natrium
D,L-α-[3-(4-Hydroxy-2-p-methylaminosulfonylanilino-5-pyrimidinyl)-ureido]-m-chlor-p-hydroxy-benzylpenicillin-Natrium
D-α-[3-(4-Hydroxy-2-p-methylsulfinylanilino-5-pyrimidinyl)-ureido]-benzylpenicillin-Natrium
D,L-α-[3-(4-Hydroxy-2-p-methylsulfinylanilino-5-pyrimidinyl)-ureido]-m-chlor-p-hydroxy-benzylpenicillin-Natrium
D-α-[3-(2-(p-Aminosulfonyl-m-hydroxy-anilino)-4-hydroxy-5-pyrimidinyl)-ureido]-p-hydroxy-benzylpenicillin-Natrium
D-α-[3-(2-(p-Aminosulfonyl-m-hydroxy-anilino)-4-hydroxy-5-pyrimidinyl)-ureido]-benzylpenicillin-Natrium

5) Arzneimittel, gekennzeichnet durch einen Gehalt an einem oder mehreren Wirkstoffen der allgemeinen Formeln I bzw. I' gemäß den Ansprüchen 1 bis 4 neben den üblichen Träger-und/oder Hilfsstoffen.

6) Verfahren zur Herstellung neuer Penicilline der allgemeinen Formeln 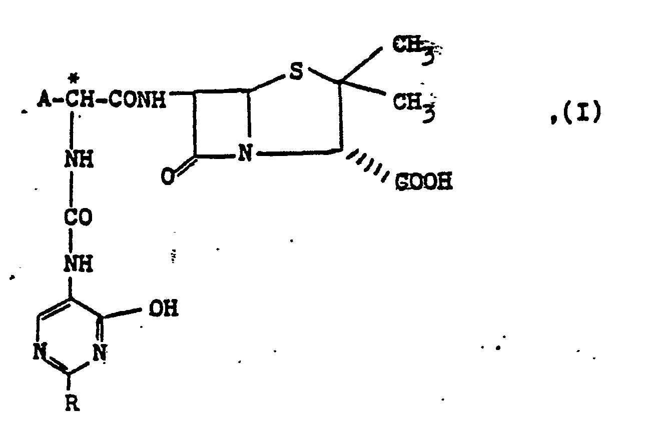 bzw. 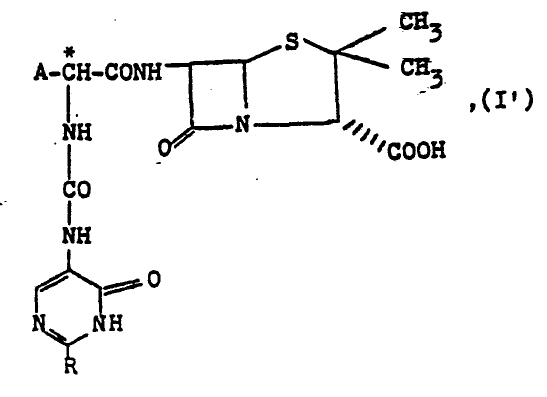 in welchen A und R die folgenden Bedeutungen besitzen:
a) A die Phenylgruppe und
R die Gruppe der allgemeinen Formel II, 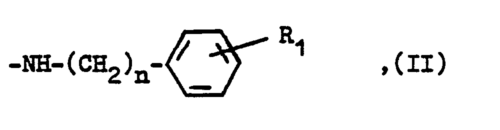 in der n=O oder 1 ist und R₁, sofern n-0 ist, die Gruppen 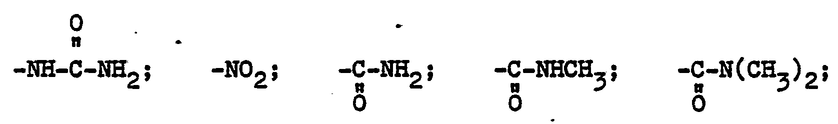 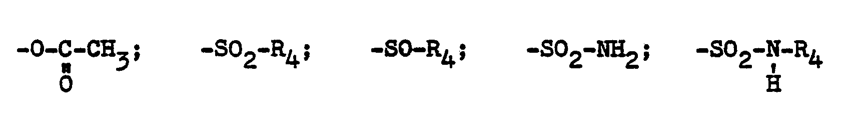 oder -SO₂-N(R₄)₂ bedeutet und, sofern n=1 ist, bedeutet es die oben genannten Gruppen und noch zusätzlich ein Wasserstoff- oder Fluoratom, die Hydroxy-, Methyl- oder Methoxygrüppe, wobei R₄ in den obigen Formeln eine Alkylgruppe mit 1 bis 4 Kohlenstoffatomen bedeutet, oder R eine Gruppe der allgemeinen Formel III, 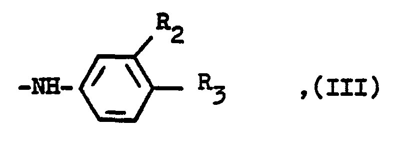 in der einer der Reste R₂ oder R₃ die Hydroxygruppe bedeutet und der andere dieser Reste eine Gruppe der Formeln 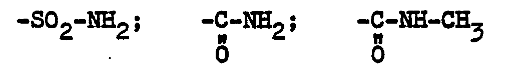 oder die beiden Reste R₂ und R₃ die Gruppe -CONH₂ darstellen, oder es bedeutet
b) A die p-Hydroxyphenylgruppe und
R die Gruppe der allgemeinen Formel II, in der n=0 oder 1 ist und R₁, sofern n-0 ist, die Gruppen der Formeln 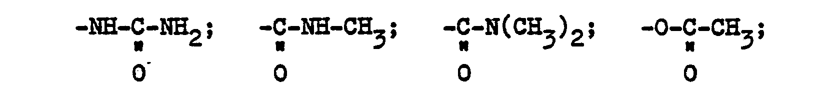 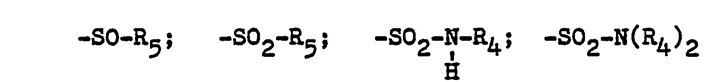 oder die meta-ständige Gruppe -SO₂-NH₂ bedeutet, und, sofern n-1 ist, bedeutet es die obengenannten Gruppen und noch zusätzlich ein Fluoratom, die Hydroxygruppe, die Nitrogruppe, die Gruppe H₂NCO-, die Gruppen -SO-CH₃, -SO₂-CH₃, oder p-H₂N-SO₂-, oder die Methyl- oder Methoxygruppe wobei in den obigen Formeln R₄ wie weiter oben angegeben definiert ist und R₅ eine Alkylgruppe mit 2 bis 4 Kohlenstoffatomen bedeutet, oder
R eine Gruppe der allgemeinen Formel III, in der einer der Reste R₂ oder R₃ die Hydroxygruppe bedeutet und der andere dieser Reste eine Gruppe der Formeln 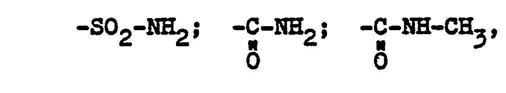 oder in der die beiden Reste R₂ und R₃ die Gruppe -CONH₂ darstellen, oder es bedeutet
c) A die 2-Thienylgruppe, die Cyclohexa-1,4-dien-1-ylgruppe, die 3,4-Dihydroxyphenyl- oder die 3-Chlor-4-hydroxyphenylgruppe und
R eine Gruppe der allgemeinen Formel II, in der n=0 oder 1 ist und R₁ ein Fluoratom, die Methyl- oder Methoxygruppe oder eine Gruppe der Formeln 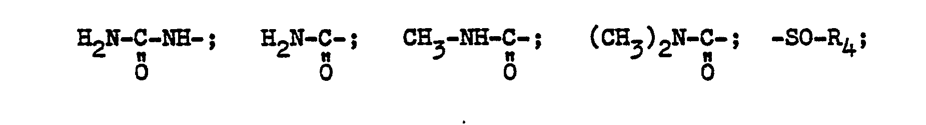 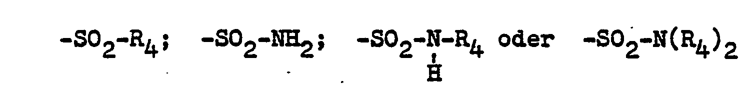 darstellt, wobei in diesen Formeln R₄ wie oben angegeben definiert ist, und R₁ auch ein Wasserstoffatom sein kann, sofern dann A die 2-Thienyl-, 3,4-Dihydroxyphenyl- oder 3-Chlor-4-hydroxyphenylgruppe bedeutet oder R₁ die Hydroxygruppe oder die Methylcarbonylgruppe, sofern A die 2-Thienylgruppe, die Cyclohexa-1,4-dien-1-ylgruppe oder die 3-Chlor-4-hydroxyphenylgruppe darstellt, oder
R eine Gruppe der allgemeinen Formel III, in der R₂ die Hydroxygruppe und R₃ die Hydroxygruppe oder die Gruppen -CONH₂, -CO-NHCH₃, -SO₂-NH₂ bedeuten, beide Reste R₂ und R₃ können auch gleichzeitig die Gruppe -CONH₂ bedeuten und von deren Salzen mit anorganischen oder organischen Basen, dadurch gekennzeichnet, daß
a) eine Verbindung der allgemeinen Formel V, 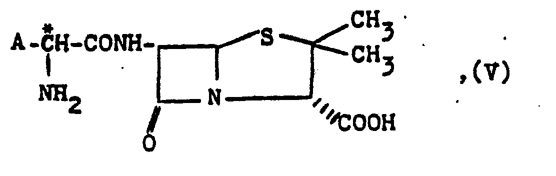 in der A wie oben definiert ist, mit einem Pyrimidinderivat der allgemeinen Formel VI, 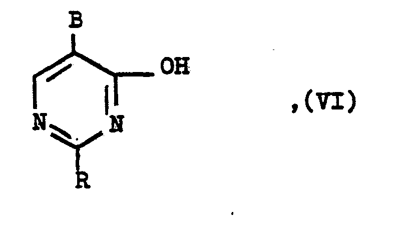 in der R wie oben definiert ist und B die Gruppe -NCO oder ein reaktives Derivat dieser Gruppe bzw. die Gruppen -NHCOC1, -NHCOBr oder 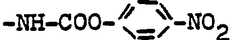 bedeutet oder mit Gemischen solcher Pyrimidine der allgemeinen Formel VI, in der B teils die eine und teils die andere der vorstehend genannten Bedeutungen besitzt, in einem Lösungsmittel und einem pH-Bereich zwischen 2,0 und 9,0 bei Temperaturen zwischen -20°C und +50°C umgesetzt wird, oder
b) eine Ureidocarbonsäure der allgemeinen Formel VII 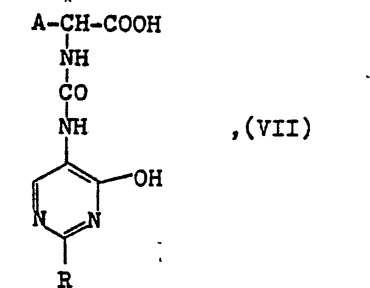 in der A und R die oben angegebenen Bedeutungen besitzen, oder ihre Salze oder reaktiven Derivate, mit 6-Aminopenicillansäure der Formel VIII, 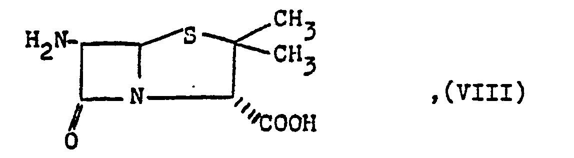 oder mit ihren anorganischen oder organischen Salzen oder mit ihren Derivaten, die in die 6-Aminopenicillansäure leicht überführbar sind, zwischen -40° und +40°C in Gegenwart eines Lösungsmittels und, gegebenenfalls, einer Base umgesetzt wird und/oder eine so erhaltene Verbindung der allgemeinen Formel I bzw. I' in ihre Salze mit anorganischen oder organischen Basen überführt wird.

7) Verfahren gemäß Anspruch 6a, dadurch gekennzeichnet, daß eine Verbindung der allgemeinen Formel V oder eines ihrer Salze mit anorganischen oder organischen Basen mit einer Verbindung der allgemeinen Formel VI,
a) in Wasser oder in mit Wasser mischbaren Lösungsmitteln in Gegenwart von Wasser in einem pH-Bereich von 6,5 bis 8,0 oder
b) in wasserfreien Lösungsmitteln oder
c) in einem Gemenge aus Wasser und mit Wasser nicht mischbaren Lösungsmitteln in einem pH-Bereich zwischen 6,5-und 8,0
umgesetzt wird, oder daß eine Verbindung der allgemeinen Formel V, in der das Wasserstoffatom der Carboxylgruppe durch eine Silylgruppe oder eine andere leicht abspaltbare Schutzgruppe ersetzt ist, mit einer Verbindung der allgemeinen Formel VI in einem wasser- und hydroxylgruppenfreien bzw. aprotischen Lösungsmittel, gegebenenfalls in Gegenwart einer Base, umgesetzt wird.

8) Verfahren gemäß Anspruch 6b, dadurch gekennzeichnet, daß als reaktive Derivate der Ureidocarbonsäuren der allgemeinen Formeln VII deren Säureanhydride, deren reaktive Ester oder reaktive Amide oder deren Säurehalogenide, als Derivate der Verbindung der Formel VIII der Diphenylmethylester, der t-Butylester, der Trimethylsilylester oder das N,O-Bis-trimethylsilylderivat verwendet werden und die Umsetzung in Gegenwart einer Base und/oder eines organischen Lösungsmittels und/oder eines Kondensationsmittels erfolgt und, gewünschtenfalls, das so erhaltene Produkt der allgemeinen Formel I, in der das Wasserstoffatom der ^{?} Carboxylgruppe durch eine abspaltbare Schutzgruppe ersetzt ist, anschließend in eine Säure der allgemeinen Formel I bzw. I' übergeführt wird.

1) Verfahren zur Herstellung neuer Penicilline der allgemeinen Formeln 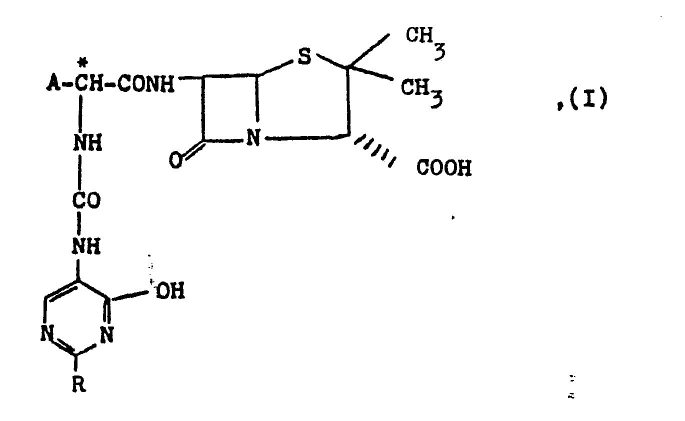 bzw. 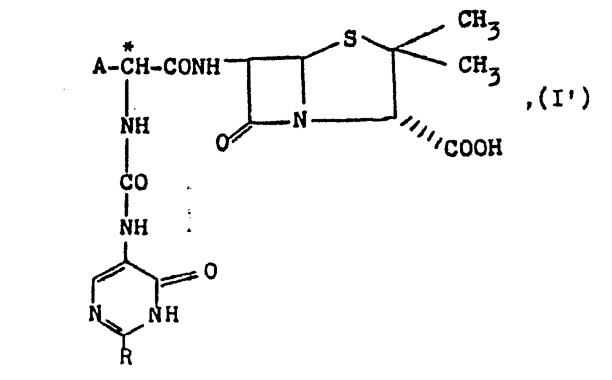 in welchen A und R die folgenden Bedeutungen besitzen:
a) A die Phenylgruppe und
R die Gruppe der allgemeinen Formel II, 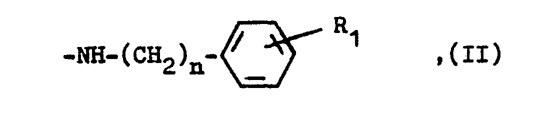 in der n=0 oder 1 ist und R₁, sofern n=0 ist, die Gruppen 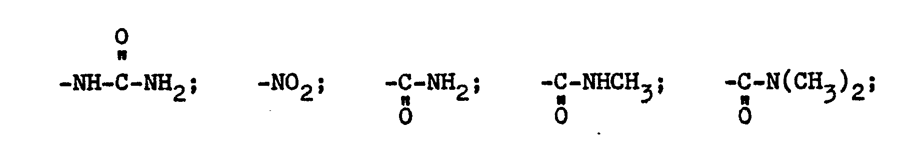 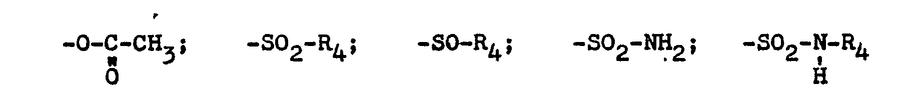 oder -SO₂-N(R₄)₂ bedeutet und, sofern n=1 ist, bedeutet es die oben genannten Gruppen und noch zusätzlich ein Wasserstoff- oder Fluoratom, die Hydroxy-, Methyl- oder Methoxygruppe, wobei R₄ in den obigen Formeln eine Alkylgruppe mit 1 bis 4 Kohlenstoffatomen bedeutet, oder R eine Gruppe der allgemeinen Formel III, 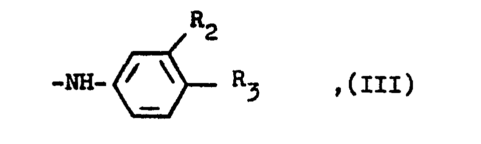 in der einer der Reste R₂ oder R₃ die Hydroxygruppe bedeutet und der andere dieser Reste eine Gruppe der Formeln 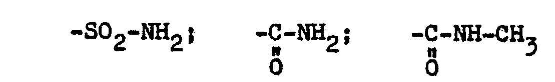 oder die beiden Reste R₂ und R₃ die Gruppe -CONH₂ darstellen, oder es bedeutet
b) A die p-Hydroxyphenylgruppe und
R die Gruppe der allgemeinen Formel II, in der n=0 oder 1 ist und R₁, sofern n=0 ist, die Gruppen der Formeln 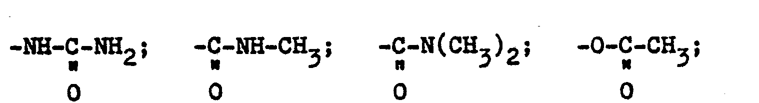 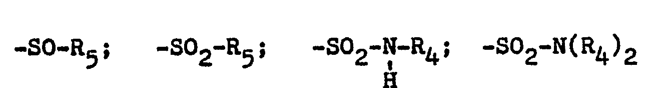 oder die meta-ständige Gruppe -SO₂-NH₂ bedeutet, und, sofern n=1 ist, bedeutet es die obengenannten Gruppen und noch zusätzlich ein Fluoratom, die Hydroxygruppe, die Nitrogruppe, die Gruppe H₂NCO-, die Gruppen -SO-CH₃, -SO₂-CH₃, oder p-H₂N-SO₂-, oder die Methyl- oder Methoxygruppe wobei in den obigen Formeln R₄ wie weiter oben angegeben definiert ist und R₅ eine Alkylgruppe mit 2 bis 4 Kohlenstoffatomen bedeutet, oder
R eine Gruppe der allgemeinen Formel III, in der einer der Reste R₂ oder R₃ die Hydroxygruppe bedeutet und der andere dieser Reste eine Gruppe der Formeln 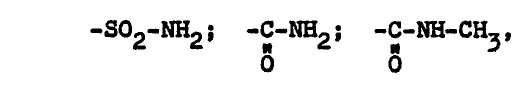 oder in der die beiden Reste R₂ und R, die Gruppe -CONH₂ darstellen, oder es bedeutet
c) A die 2-Thienylgruppe, die Cyclohexa-1,4-dien-1-ylgruppe, die 3,4-Dihydroxyphenyl- oder die 3-Chlor-4-hydroxyphenylgruppe und
R eine Gruppe der allgemeinen Formel II, in der n=O oder 1 ist und R₁ ein Fluoratom, die Methyl- oder Methoxygruppe oder eine Gruppe der Formeln 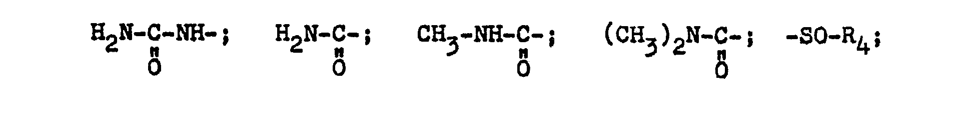 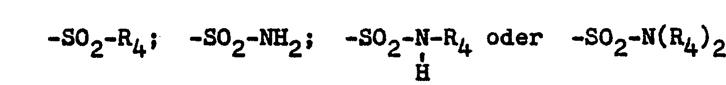 darstellt, wobei in diesen Formeln R₄ wie oben angegeben definiert ist, und R₁ auch ein Wasserstoffatom sein kann, sofern dann A die 2-Thienyl-, 3,4-Dihydroxyphenyl- oder 3-Chlor-4-hydroxyphenylgruppe bedeutet oder R₁ die Hydroxygruppe oder die Methylcarbonylgruppe, sofern A die 2-Thienylgruppe, die Cyclohexa-1,4-dien-1-ylgruppe oder die 3-Chlor-4-hydroxyphenylgruppe darstellt, oder
R eine Gruppe der allgemeinen Formel III, in der R₂ die Hydroxygruppe und R₃ die Hydroxygruppe oder die Gruppen -CONH₂, -CO-NHCH₃, -SO₂-NH₂ bedeuten, beide Reste R₂ und R₃ können auch gleichzeitig die Gruppe -CONH₂ bedeuten und von deren Salzen mit anorganischen oder organischen Basen, dadurch gekennzeichnet, daß
a) eine Verbindung der allgemeinen Formel V, 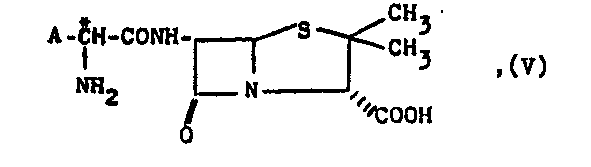 in der A wie oben definiert ist, mit einem Pyrimidinderivat der allgemeinen Formel VI, 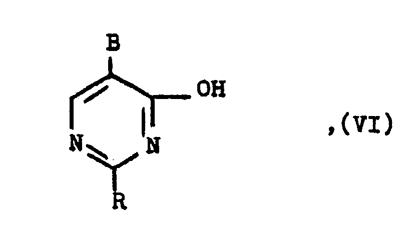 in der R wie oben definiert ist und B die Gruppe -NCO oder ein reaktives Derivat dieser Gruppe bzw. die Gruppen -NHCOC1, -NHCOBr oder 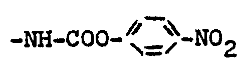 bedeutet oder mit Gemischen solcher Pyrimidine der allgemeinen Formel VI, in der B teils die eine und teils die andere der vorstehend i genannten Bedeutungen besitzt, in einem Lösungsmittel und einem pH-Bereich zwischen 2,0 und 9,0 bei Temperaturen zwischen -20°C und +50°C umgesetzt wird, oder
b) eine Ureidocarbonsäure der allgemeinen Formel VII 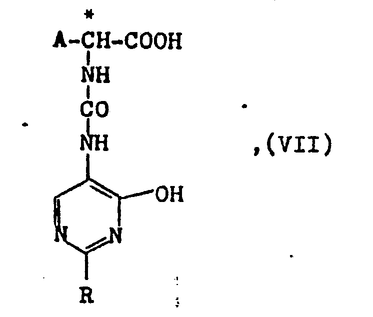 in der A und R die oben angegebenen Bedeutungen besitzen, oder ihre Salze oder reaktiven Derivate, mit 6-Aminopenicillansäure der Formel VIII, 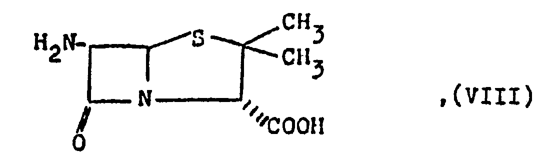 oder mit ihren anorganischen oder organischen Salzen oder mit ihren Derivaten, die in die 6-Aminopenicillansäure leicht überführbar sind, zwischen -40° und +40°C in Gegenwart eines Lösungsmittels und, gegebenenfalls, einer Base umgesetzt wird und/oder eine so erhaltene Verbindung der allgemeinen Formel I bzw. I' in ihre Salze mit anorganischen oder organischen Basen überführt wird.

2) Verfahren gemäß Anspruch 1a, dadurch gekennzeichnet, daß eine Verbindung der allgemeinen Formel V oder eines ihrer Salze mit anorganischen oder organischen Basen mit einer Verbindung der allgemeinen Formel VI,
a) in Wasser oder in mit Wasser mischbaren Lösungsmitteln in Gegenwart von Wasser in einem pH-Bereich von 6,5 bis 8,0 oder
b) in wasserfreien Lösungsmitteln oder
c) in einem Gemenge aus Wasser und mit Wasser nicht mischbaren Lösungsmitteln in einem pH-Bereich zwischen 6,5-und 8,0
umgesetzt wird, oder daß eine Verbindung der allgemeinen Formel V, in der das Wasserstoffatom der Carboxylgruppe durch eine Silylgruppe oder eine andere leicht abspaltbare Schutzgruppe ersetzt ist, mit einer Verbindung der allgemeinen Formel VI in einem wasser- und hydroxylgruppenfreien bzw. aprotischen Lösungsmittel, gegebenenfalls in Gegenwart einer Base, umgesetzt wird.

3) Verfahren gemäß Anspruch 1b, dadurch gekennzeichnet, daß als reaktive Derivate der Ureidocarbonsäuren der allgemeinen Formeln VII deren Säureanhydride, deren reaktive Ester oder reaktive Amide oder deren Säurehalogenide, als i Derivate der Verbindung der Formel VIII der Diphenylmethylester, der t-Butylester, der Trimethylsilylester oder das N,0-Bis-trimethylsilylderivat verwendet werden und die Umsetzung in Gegenwart einer Base und/oder eines organischen Lösungsmittels und/oder eines Kondensationsmittels i erfolgt und, gewünschtenfalls, das so erhaltene Produkt der allgemeinen Formel I, in der das Wasserstoffatom der Carboxylgruppe durch eine abspaltbare Schutzgruppe ersetzt ist, anschließend in eine Säure der allgemeinen Formel I bzw. I' übergeführt wird.
